# EUROPÄISCHE PATENTSCHRIFT

(11) **EP 2 409 146 B1**
(45) Veröffentlichungstag und Bekanntmachung des Hinweises auf die Patenterteilung: **06.03.2013**
(21) Anmeldenummer: 09796957.0
(22) Anmeldetag: 14.12.2009
(51) Int. Cl.: G01N 33/14, G01N 33/00, G01N 33/02, G01N 35/00

(54) **Verfahren und Vorrichtung zur Bestimmung eines Fremdstoffgehalts in einer Matrix**
Method and device for determining a foreign substance content in a matrix
Procédé et dispositif pour déterminer une teneur en matière étrangère dans une matrice

(30) Priorität: 19.03.2009 DE 102009013534
(43) Veröffentlichungstag der Anmeldung: 25.01.2012
(73) Patentinhaber: Crinotec GmbH, 72070 Tübingen (DE)
(72) Erfinder: FIEDLER, Andreas, 72072 Tübingen (DE)
(74) Vertreter: Kaufmann, Ursula Josefine
(86) Internationale Anmeldenummer: PCT/EP2009/008936
(87) Internationale Veröffentlichungsnummer: WO 2010/105650

(56) Entgegenhaltungen:
- EP-A1- 0 993 241
- EP-A1- 1 840 557
- EP-B1- 1 285 266
- WO-A1-83/00932
- DE-A1-102004 047 822
- US-A- 3 873 273
- US-A1- 2003 148 415

## Beschreibung

Die Erfindung betrifft Anspruchs 1, sowie ein Verfahren zum Betreiben einer solchen Vorrichtung eine Vorrichtung zur Bestimmung eines Fremdstoffgehalts in Form von Sulfit in einer Matrix eines Festen oder Flüssigen Lebensmittels gemäß dem Oberbegriff des unabhängigen.

In verschiedenen Anwendungsgebieten ist es von Interesse, den Gehalt einer in einer Matrix, z.B. einer flüssigen Probe frei und/oder an Inhaltsstoffe gebunden vorhandenen Fremdstoff komponente zu kennen. Eine solche Komponente ist z.B. Schwefeldioxid (SO₂), deren Gehalt etwa in Wein bestimmt werden soll. Aber auch in Fruchtsäften oder in anderen Nahrungsmitteln, auch in fester Konsistenz, ist SO₂ eine aus gesundheitlichen Gründen nicht zu vernachlässigende Komponente.

Darüber hinaus findet SO₂ in der Umweltanalytik Beachtung, z. B. bei Kontrollsystemen der Abluft, der Rauchgasentschwefelung, der MAK Bestimmung, etc.

Für viele Einsatzbereiche von SO₂ hat der Gesetzgeber inzwischen Grenzwerte erlassen, die von der Industrie, den Erzeuger und Abfüllbetrieben eingehalten und folglich regelmäßig kontrolliert werden müssen.

Ein Verzicht auf SO₂ z. B. bei der Weinherstellung ist häufig nur bis zu einem gewissen Maß möglich, denn SO₂ wird z. B. beim "Ausschwefeln" der Weinfässer eingesetzt, dient aber auch als Konservierungsstoff und wird allgemein als unverzichtbare Komponente für die qualitativ hochwertige Weinherstellung angesehen. Dennoch hat SO₂ gesundheitlich nachteilige Nebenwirkungen, die sich z. B. durch starke Kopfschmerzen nach dem Konsum von stark SO₂-haltigen Weinen manifestieren.

Darüber hinaus gibt es insbesondere bei Wein eine ganze Reihe von Weinfehlern und Weinkrankheiten, die durch unerwünschte oder zu hohe Konzentrationen von Komponenten verursacht werden.

Hierzu zählt z. B. der durch Essigsäure hervorgerufene Essigstich, der u. a. durch Schwefelwasserstoff hervorgerufene Böckser, der durch u. a. 2,4,6-Trichlor-Anisol hervorgerufene Kork-Muffton oder der durch 3-Methyloctano-4-Lacton hervorgerufene Holzton.

Wegen weiterer Informationen zu Weinfehler und Weinkrankheiten verursachenden Komponenten siehe z.B. Römpp Lexikon Chemie, 10. Auflage, 1999, Georg Thieme Verlag, Stuttgart.

Im Wein kommt Schwefeldioxid bzw. die schweflige Säure sowohl frei als auch an verschiedene Inhaltsstoffe gebunden vor. Der gesamte Gehalt an schwefliger Säure wird durch die Summe aller Zustandsformen repräsentiert, der auf bestimmte Maximalwerte hin überwacht werden muss. Die Entstehung von gebundenem SO₂ beruht im Wesentlichen auf der so genannten Bisulfit-Addition an Aldehyde und Ketone.

Aus den gesetzlichen Untersuchungsvorschriften sowie entsprechenden Handbüchern für Winzer ist eine ganze Reihe von Messverfahren bekannt, um die freie schweflige Säure oder das freie SO₂ zu bestimmen. Man unterscheidet dabei so genannte Referenzverfahren und Schnellverfahren. Die bekannten Referenzverfahren sind sehr zeitaufwändig, erfordern entsprechenden apparativen Aufwand und sind entsprechend teuer. Die Analysen bei Schnellverfahren werden durch viele Störparameter ungenau und schlecht reproduzierbar. Ein Problem ist dabei die in Wein sehr häufig vorhandene Ascorbinsäure, die z. B. bei zu den anerkannten Schnellverfahren zählenden iodometrischen Verfahren, die zu den titrimetrischen Verfahren zählen, fälschlich auch wie schweflige Säure erfasst wird. Zur Ermittlung der Konzentration der freien schwefligen Säure muss die Ascorbinsäure gesondert bestimmt und vom erhaltenen Wert abgezogen werden.

Neben der lodometrie ist es auch bekannt, SO₂ z.B. durch Colorimetrie, Oxidationsverfahren, Gasmesselektroden, enzymatische Verfahren oder mittels Gaschromatographie zu bestimmen.

Damit bei den bekannten Verfahren auch die gebundene schweflige Säure mit bestimmt werden kann, wird diese durch Verseifung mit Lauge oder in der Hitze durch ein anerkanntes Referenzverfahren wie Destillation mit starken Säuren freigesetzt, was weitere, kostspielige und zeitaufwendige Verfahrensschritte beinhaltet, die zudem quantitativ nicht immer hinreichend zuverlässig sind.

Bei der Gaschromatographie wird z.B. mit Hilfe eines Flammenphotometers oder eines elektrolytischen Hall-Detektors die SO₂-Konzentration im Gasraum über der flüssigen Probe gemessen, wobei unter der Anwendung des Henry'schen Gesetzes dann die Konzentration der schwefligen Säure in Lösung berechnet wird. Das Henry'sche Gesetz, auch Henry'sches Absorptionsgesetz genannt, bringt zum Ausdruck, dass der Dampfdruck eines gelösten Stoffes proportional zu seinem Molenbruch in einer ideal verdünnten Lösung ist. Die Proportionalität wird durch eine empirische, temperaturabhängige Henrykonstante angegeben. Obwohl die Messung mit anschließender Rechnung zum zufrieden stellenden Ergebnis führt, wird die Verwendung eines Hall-Detektors oder eines Flammenphotometers jedoch als begrenzend für das angegebene Verfahren angegeben. Sowohl die Kosten als auch die erforderliche experimentelle Erfahrung für den Einsatz der angegebenen Detektoren begrenzen ihre Anwendung auf große Fachbetriebe. Für kleinere Winzer, Abfüller oder Analyselaboratorien ist das bekannte Verfahren nicht rentabel einzusetzen.

Die insoweit beschriebenen Verfahren der Schwefeldioxidanalyse erfordern also einen großen zeitlichen und apparativen Aufwand, wobei besonders von Nachteil ist, dass andere Komponenten mitbestimmt werden, so dass diese gesondert gemessen und vom erhaltenen Messwert abgezogen werden müssen.

Aus der EP 1285 266 B1 ist eine vereinfachte Vorrichtung und ein Verfahren zur Messung des SO₂-Gehalts in Wein bekannt, bei dem der Wein durch ein Trägergas begast wird, das im Kurzschluss durch den Wein geleitet wird. Dabei stellt sich ein Gleichgewichtszustand ein, so dass die Konzentration des in der Gasphase befindlichen SO₂ mittels einer elektrochemischen Zelle erfasst und ausgewertet werden kann. Während der Probenvorbereitung kann die Gasführung automatisiert erfolgen. Das Verfahren liefert hinreichend gute Ergebnisse.

Aus der EP1 840 557 ist eine Vorrichtung bekannt, mit der die Zusammensetzung von Flüssigkeiten bestimmt werden kann. Reagenzien werden in einem Reagenzienbereich zugeführt und eine Probe in einem Reaktionsbereich dosiert mit den Reagenzien versetzt, welches Konstituenten in einen Gasraum freisetzt oder eine flüchtige Verbindung mit diesen bildet. Die Zusammensetzung kann quantitativ und qualitativ bestimmt werden, indem Absorptionsspektren des Gases im Gasraum aufgenommen und das Absorptionsverhalten des Gases analysiert wird.

Die US 3 873 273 A offenbart eine automatisierte Anlage zum Testen von Blutproben. Das Vorhandensein der Blutproben wird ermittelt, und es werden optische Transmissionseigenschaften der Blutproben untersucht, die mit einem Reagenz versetzt sind.

Die WO 83/00932 A1 zeigt ein System zum Speichern und Abgeben von Reagenzien. Reagenzienbehälter sind jeweils in Behälteraufnahmen eines Trägers eingeschraubt, wobei beim Einschrauben eine Nadel in der Behälteraufnahme ein Durchstechseptum durchsticht.

Es ist die Aufgabe der Erfindung, eine weiter verbessertes Verfahren und eine entsprechende Vorrichtung zu schäften, mit der auch weniger geübte Personen vor Ort einen Fremdstoff-Nachweis in einer Matrix führen können.

Diese Aufgabe wird erfindungsgemäß durch die Merkmale der unabhängigen Ansprüche gelöst. Die weiteren Ansprüche, die Zeichnung und die Beschreibung beschreiben günstige Ausgestaltungen der Erfindung.

Entsprechend wird eine Vorrichtung zur Bestimmung eines Gehalts wenigstens eines Fremdstoffs in Form von Sulfit in einer Matrix eines festen oder flüssigen Lebensmittels gemäβ Anspruch 1 vorgeschlagen. Vorteilhaft sind der in Anspruch genamte Reagenzienbehälter und/oder der Probenbehälter vorkonfektioniert, so dass sie bereits mit chemischen Komponenten vorbefüllt sind, die in Art und Menge genau aufeinander abgestimmt sind. Besonders bevorzugt sind sie so ausgebildet, dass sie verwechslungssicher in die Vorrichtung einsetzbar sind. Der Anwender braucht lediglich die zu untersuchende, gegebenenfalls Fremdstoff enthaltene Matrix in vorbestimmter Menge zuzugeben. So kann etwa ein Winzer den Sulfitgehalt (SO₂-Gehalt) im Wein vor Ort im Weinkeller prüfen. SO₂ stellt dabei den Fremdstoff dar, der Wein die Matrix. Analysiert werden können jedoch auch z.B. geschwefeltes Trockenobst, das zerkleinert und z.B. mit Wasser versetzt wird, Fruchtsäfte, Bier und dergleichen. Als Lebensmittel sind Stoffe und Produkte zu verstehen, die vom Menschen zum Zwecke der Ernährung oder des Genusses über den Mund, gegebenenfalls nach vorheriger Zubereitung, aufgenommen werden. Allgemein sind dabei Nahrungsmittel, Genussmittel, Lebensmittelzusatzstoffe und Nahrungsmittelergänzungsmittel eingeschlossen. Ebenso können Futterstoffe für Tiere darunter subsumiert werden.

Im Folgenden bezeichnet SO₂ den Sulfitgehalt, der in gelöster und in gebundener Form in der Matrix vorliegen kann. Gelöstes SO₂ wird auch als freies SO₂ bezeichnet. Die Vorrichtung kann günstigerweise sowohl zum Nachweis von freiem SO₂ alleine als auch zum Nachweis des gesamten Gehalts an SO₂ eingesetzt werden, welches in dem Lebensmittel in gelöster und in gebundener Form vorliegt.

Der Reagenzienbereich umfasst wenigstens eine Sensoreinrichtung die ein Vorhandensein oder ein Nichtvorhandensein des wenigstens einen Reagenzes und/oder das Vorhandensein des Reagenzeinbehälters in dem Reagenzienbereich automatisch detektiert. Ebenso kann zusätzlich die Reagenzienart im Reagenzienbehälter detektiert werden. Die Sensoreinrichtung kann z.B. eine Lichtschranke sein, die eine Codierung auf dem Reagenzienbehälter erfasst, oder ein Barcode, eine RFID-Codierung (Radio Frequency IDentification), ein Hebel, ein Mikroschalter und dergleichen.

Der Reagenzienbereich weist eine Aufnahme auf, die einen Reagenzienbehälter aufnimmt, vorzugsweise kann der Reagenzienbehälter in die Aufnahme ganz eintauchen. Der Reagenzienbehälter ist in ein Trägergehäuse einsetzbar und mit dem Trägergehäuse in dem Reagenzienbereich einsetzbar. Dadurch kann der Reagenzienbehälter leichter gehandhabt werden und mit dem Trägergehäuse zusammen leicht wieder aus der Aufnahme entfernt werden. Das Trägergehäuse kann zum hermetischen Verschließen der Aufnahme ausgebildet sein, was die Sicherheit der Vorrichtung erhöht. Der Reagenzienbehälter kann z.B. als Durchstech-Ampulle mit einem Durchstich-Septum als Verschluss ausgebildet sein, oder mit einer aufgeschweißten oder aufgeklebten Metall- oder Kunststofffolie verschlossen sein.

Der Reagenzienbereich weist zum Anschließen des Reagenzienbehälters ein Nadelsystem auf, auf das der Reagenzienbehälter aufsteckbar ist. Vorzugsweise kann das Nadelsystem wenigstens zwei Hohlnadeln umfassen, die in den Reagenzienbehälter eintauchen. Eine Nadel stellt vorzugsweise den Eingang des Reagenzienbehälters dar, der z.B. mit einer Fördereinrichtung, z.B. einer Pumpe oder einem Reservoir, verbunden sein kann, die andere stellt einen Ausgang dar, der z.B. mit dem Reaktionsbereich verbunden ist. Die Nadeln können dabei auch unterschiedlich tief in den Reagenzienbehälter ragen. Die längere Nadel kommt zuerst mit dem Innenraum des Reagenzienbehälters in Kontakt. Bevorzugt stellt die längere Nadel den Ausgang für das Reagenz dar. Dadurch kann ein etwaiger Überdruck im Reagenzienbehälter beim Anschließen des Reagenzienbehälters zuverlässig das Reagenz in die gewünschte Richtung zum Reaktionsbereich hin fördern, wenn dieser über die längere Nadel an den Reagenzienbereich angeschlossen ist. Das Nadelsystem kann dabei parallel nebeneinander angeordnete Nadeln umfassen, oder die Nadeln können koaxial angeordnet sein, z.B. kann eine kürzere Nadel in einer längeren Nadel stecken. Alternativ kann auch eine Hohlnadel mit einem innenliegenden Schlauch vorgesehen sein.

Zusätzlich oder alternativ kann auch eingangsseitig ein Rückschlagventil vorgesehen sein, so dass sichergestellt ist, dass das Reagenz den Reagenzienbehälter nur in einer gewünschten Richtung verlassen kann, selbst wenn die Nadeln gleich weit in den Reagenzienbehälter ragen sollten. Weiterhin kann vorteilhaft vorgesehen sein, dass die Nadeln als Baueinheit so ausgebildet sind, dass die Nadeln leicht ausgetauscht werden können. Die Nadeln können dazu in einen Block integriert sein, der entsprechende Anschlussmöglichkeiten etwa für ein Fördermittel und für eine Verbindung zum Reagenzienbereich aufweist.

Vorteilhaft kann der Reaktionsbereich zum Anschließen eines Probenbehälters ein Zuführsystem aufweisen, durch welches wenigstens das Reagenz zuführbar ist. Bevorzugt kann der freigesetzte Fremdstoff durch das Zuführsystem abführbar sein und z. B. zum Sensor geleitet werden. Das Zuführsystem kann als Nadelsystem ausgebildet sein. Denkbar ist jedoch auch, Schläuche oder Röhrchen vorzusehen. Dabei ist eine parallel nebeneinander liegende Anordnung von Nadeln und/oder Schläuchen und/oder Röhrchen wie auch eine koaxiale Anordnung möglich. Das Zuführsystem kann bevorzugt durch einen Stopfen geführt sein, welcher als Spritzschutz dienen kann und gegebenenfalls den Probenbehälter beim Anschließen an den Reaktionsbereich auch abdichten kann. Vorzugsweise kann der Probenbehälter auf einen konusförmigen Zapfen aufgesteckt werden, aus dem das Zuführsystem mit dem Stopfen ragt. Am Zapfen kann ein Dichtring oder eine Dichtlippe zum Verschließen des aufgesteckten Probenbehälters angeordnet sein. Dadurch kann der Probenbehälter einfach und sicher an den Reaktionsbereich angedockt werden.

Vorteilhaft kann ein Sensor in einen Gasraum des Probenbehälters einführbar sein. Dies kann alternativ oder zusätzlich zu einem Sensor in einem vom Probenbehälter entfernten Sensorbereich vorgesehen sein.

Der Reaktionsbereich kann eine Heiz- und/oder Kühleinrichtung aufweisen. Dadurch kann die Nachweisreaktion vorteilhaft geeignet unterstützt werden. Das Erwärmen kann z.B. durch externe Komponenten, wie etwa eine Heizwendel in der Probe, Heizen des Leitungssystems, Heizung durch Strahlung (z.B. Infrarot, Mikrowellen), einen Heizmantel um den Probenbehälter, Zugabe von Stoffen, die beim Lösen Wärme freisetzen etc. erfolgen. Ebenso ist zusätzlich oder alternativ ein Einsatz von Reagenzien im Probenbehälter möglich, die aufgrund ihrer Aggressivität keine zusätzliche Erwärmung benötigen, oder die selbst Wärme beim Kontakt mit einem Stoff oder der Probe freisetzen. Eine Kühlung kann durch externe Komponenten erfolgen oder durch Zugabe von Stoffen, die beim Lösen Wärme verbrauchen. Denkbar ist auch eine ungekühlte Reaktion.

Bevorzugt kann der Sensorbereich einen elektrochemischen Sensor umfassen. Günstigerweise kann der elektrochemische Sensor eine Referenzelektrode umfassen mit einem chloridfreien Redoxsystem. Vorzugsweise kann die Referenzelektrode ein Pb/PbS04-System umfassen. Damit kann ein Referenzpotential für den elektrochemischen Sensor verfügbar gemacht werden, auf das das Messpotential bezogen werden kann. Das Referenzelektrodensystem ist günstigerweise chloridfrei und weist eine Langzeitstabilität des Systems von mindestens einem Jahr im eingebauten Zustand auf. Der Sensor erlaubt eine einfache und unkritische Handhabung und ist insbesondere stabil im sauren Milieu. Es erfolgt insbesondere kein "Ausbluten" des Systems durch Konzentrationsunterschiede. Eine Störung oder Vergiftung der fremdstoffsensitiven Arbeitselektrode des elektrochemischen Sensors kann vermieden werden. Soll, je nach chemischem System, im alkalischen Bereich gearbeitet werden, kann das Referenzelektrodensystem geeignet ausgewählt werden.

Alternativ oder zusätzlich kann der Sensorbereich einen photometrischen Sensor umfassen und/oder andere übliche Detektionsverfahren bereitstellen.

Gemäß einer günstigen Weiterbildung der Vorrichtung kann eine Trägergasleitung zwischen Reaktionsbereich und Sensorbereich in einen ersten Leitungszweig zum Zuführen von Trägergas zum Sensorbereich und einen zweiten Leitungszweig zum Umgehen des Sensorbereichs aufgeteilt sein. Bei Stoffen, wie etwa das Reagenz, welche eine Membran oder eine darauf reaktive Schicht des Sensors austrocknen können, kann eine übermäßige Exposition der Membran vermieden werden, ohne die Messgenauigkeit zu verschlechtern. Ferner können die Strömungseigenschaften optimiert werden. Dadurch, dass der Gasstrom nicht direkt an der Membran vorbeigeführt werden muss, können sich vorteilhafte diffusionskontrollierte, quasi statische Verhältnisse am Sensorbereich bzw. an der Membran ausbilden. Schwankungen im Gasdurchfluss können dadurch kompensiert werden, was sich positiv auf die Messgenauigkeit auswirkt. Dies ist vorteilhaft, wenn das Trägergas zeitlich unterschiedliche Konzentrationen des Analyten aufweist und so am Sensor zeitlich variable Konzentrationen anliegen.

Gemäß einem weiteren Aspekt der Erfindung wird in Anspruch 9 ein auswechselbarer Reagenzienbehälter für die genannte Vorrichtung zur Bestimmung eines Gehalts an Fremdstoff in Form von Sulfit in einer Matrix eines festen oder flüssigen Lebensmittels gemäβ Anspruch 1 vorgeschlagen, der gebrauchsfertig mit einer definierten Menge wenigstens eines Reagenzes für die Verwendung in der Vorrichtung hergerichtet und an die Aufnahme des Reagenzbereichs verwechstungssicher und auswechselbar anschließbar ist. Es können eine oder mehrere Codierungen vorgesehen sein zum Nachweis der Anwesenheit in der Vorrichtung und/oder der Art des enthaltenen Reagenzes. Das wenigstens eine Reagenz kann eine Säure sein, vorzugsweise wenigstens eine Säure aus der Gruppe von Schwefelsäure, Phosphorsäure, Salzsäure.

Je nach zu untersuchender Art der Matrix können gegebenenfalls andere anorganische oder organische Säuren und/oder andere Konzentrationsbereiche der Säuren günstig sein. Alternativ handelt es sich bei dem Reagenz erfindungsgemäβ um ein alkalisches Reagenz, wenn im alkalischen Bereich gearbeitet werden soll. So ist beim SO₂-Nachweis in Wein z.B. auch eine alkalische Hydrolyse zum Aufschließen von Bisulfit-Addukten mit einem anschließenden Austreiben des SO₂ im sauren Milieu denkbar. In beiden Füllen ist das Reagenz aber in Art und Menge genau auf einen vor konfektionierten Probenbehälter abgestimmt, um den Gehalt des Fremdstoffs in der Matrix zu bestimmen.

Vorteilhaft kann ein Korrosionsinhibitor im Reagenzienbehälter enthalten sein. So kann beim Sulfit-Nachweis im Wein ein 50-85%iges Schwefelsäuregemisch durch Eisen(III)-Salze derart modifiziert werden, dass die Hohlnadeln des Nadelsystems im Reagenzienbereich chemisch nicht oder nur vernachlässigbar angegriffen werden. Neben Eisen(III)-Salzen sind auch andere Salze wie z.B. von Kupfer, Wolfram, Molybdän geeignet, z.B.

Al₂(SO₄)₃

MnSO₄*1 aq

ZnSO₄*7 aq

FeSO₄*7aq

CoSO₄*7 aq

H₃[P(Mo₃O₁₀)₄] * x H₂O

NiSO₄*6 H₂O

SnCl₂*2H₂O

Cu SO₄*5 aq

Fe₂O₁₂S₃*x aq

Bi₂(CO₃)₃

Na₂MoO₄*2 H₂O

Na₂WO₄*2 H₂O

VOSO₄*5H₂O.

Werden mehrere Reagenzien in der Vorrichtung verwendet, etwa bei der Bestimmung von freiem SO₂ und gebundenem SO₂ in Wein, können die verschiedenen Reagenzien mit dem Inhibitor versetzt werden, um eine definierte Inhibitorkonzentration aufrecht zu erhalten.

Weiterhin wird ein auswechselbarer Probenbehälter vorgeschlagen, der für eine Vorrichtung zur Bestimmung eines Gehalts an Fremdstoff in einer Matrix eines festen oder flüssigen Lebensmittels hergerichtet und an das Anschlusssystem des Reaktionsbereichs auswechselbar anschließbar ist, wobei wenigstens ein Mitglied der Gruppe enthalten ist (a) ein Adduktbildner in dosierter Menge, der auf den nachzuweisenden Fremdstoff abgestimmt ist und der zur Bindung eines etwaigen gelösten Fremdstoffs in der Matrix dient; (b) ein Substrat, das sich in Kontakt mit einem Reagenz und/oder einer Matrix auflöst, und (c) eine chemische Komponente mit positiver Lösungsenthalpie, so dass die chemische Komponente beim Lösen in der Matrix Wärme aufnimmt. Der Probenbehälter allein ist jedoch nicht Teil der Erfindung.

Der Probenbehälter ist insbesondere in seinen Abmessungen auf die Vorrichtung und die verwendeten Reagenz- und Probenmengen abgestimmt. Fehlmessungen durch falsche Dosierungen können so vermieden werden.

Günstigerweise kann ein Adduktbildner in dosierter Menge enthalten sein. Der Adduktbildner hat die Aufgabe, den etwaigen gelösten Fremdstoff in der Matrix zu binden. So kann erreicht werden, dass die ehemals gelösten, d.h. "freien", Anteile des Fremdstoffs in der Matrix und die ehemals chemisch gebundenen Anteile des Fremdstoffs in der Matrix später praktisch gleichzeitig freigesetzt werden können.

Günstige Adduktbildner sind insbesondere Moleküle mit Aldehyd- und/oder Ketofunktionen, gegebenenfalls auch in Ringform (z.B. Zucker). Alternativ können auch Adsorbentien eingesetzt werden, die den Fremdstoff an ihrer Oberfläche binden können. Alternativ können auch sonstige Substanzen eingesetzt werden, die eine Rückhaltung des Fremdstoffs bewirken, z.B. eine ölige Schicht, Polymerisation bei Probenzugabe (Weinzugabe) und/oder Säurezugabe etc. Eine Polymerisation kann z.B. durch Kondensation von Benzylalkohol oder seinen Estern mit starker Säure, d.h. Zugabe von Benzylalkohol zur Probe, bewirkt werden, wobei beim Einleiten der Säure die Polymerbildung eintritt.

Vorzugsweise kann der Adduktbildner auf den nachzuweisenden Fremdstoff, also Sulfit (SO₂) , abgestimmt sein. Bei dem Nachweis von SO₂ ist als Adduktbildner Brenztraubensäure oder ein Salz der Brenztraubensäure, vorzugsweise ein Natriumsalz der Brenztraubensäure, bevorzugt.

Es kann im Probenbehälter zusätzlich oder alternativ ein Substrat enthalten sein, das eine Substanz umfasst, die zur Ausbildung eines Netzwerks befähigt ist, insbesondere eines dreidimensionalen Netzwerks, wie beispielsweise Agar oder Gelatine. Das Substrat kann sich in Kontakt mit einem Reagenz und/oder einer Matrix, auflösen und für die Durchmischung von Reagenz und Adduktbildner sorgen. Die Substanz kann z.B. ein komplexbildendes Polymer, ein Chelatbildner oder ein Gel sein. Das Substrat kann insbesondere durch die Substanz gebildet sein. Der Fachmann wird abhängig von den konkreten Rahmenbedingungen und Materialien ein geeignetes Substrat wählen. Durch die Auflösung des Substrats bei Kontakt mit einem Reagenz und/oder einer Matrix kann vorteilhaft eine Durchmischung von Probe, d.h. Matrix mit Fremdstoff, und dem Adduktbildner und/oder dem Reagenz erreicht werden. Die Analysezeiten können dadurch vorteilhaft verkürzt werden. Denkbar sind jedoch auch alternativ oder zusätzlich eine Durchmischung mittels Rühren, Vibration und dergleichen.

Vorteilhaft kann zusätzlich oder alternativ eine chemische Komponente mit positiver Lösungsenthalpie im Probenbehälter enthalten sein. Dadurch kann ein Abkühlen des Proben behälters insbesondere beim Nachweis von freiem SO₂ erreicht werden.

Gemäß einem weiteren Aspekt der Erfindung werden in den Ansprüchen 12 und 13 Sets vorgeschlagen aus einem derartigen Reagenzienbehälter und einem Probenbehälter zur Verwendung in der genannten Vorrichtung zur Bestimmung eines Gehalts wenigstens eines Fremdstoffs in Form von Sulfit in einer Matrix eines festen oder flüssigen Lebensmittels. Vorteilhaft kann der Reagenzienbehälter eine Säure zum Freisetzen des gebundenen Fremdstoffs und der Probenbehälter einen auf einen nachzuweisenden Fremdstoff abgestimmten Adduktbildner enthatten. Der Reagenzienbehälter ist gebrauchsfertig mit einem geeigneten Reagenz befüllt, um den Fremdstoff in einer Matrix nachzuweisen. Der Probenbehälter ist ebenso gebrauchsfertig befüllt und kann wenigstens ein Mitglied der Gruppe enthalten
(a) ein Adduktbildner in dosierter Menge, der auf den nachzuweisenden Fremdstoff abgestimmt ist;
(b) ein Substrat, das sich in Kontakt mit einem Reagenz und/oder einer Matrix auflöst;
(c) eine chemische Komponente mit positiver Lösungsenthalpie. Optional kann auch eine Erwärmung durch eine Komponente mit negativer Lösungsenthalpie oder Reaktionsenthalpie erfolgen.

Es wird in Anspruch 14 ein Verfahren zum Betreiben der genannten Vorrichtung zur Bestimmung eines Gehalts wenigstens eines Fremdstoffs in Form von Sulfit in einer Matrix eines festen oder flüssigen Lebensmittels vorgeschlagen, bei dem ein ursprünglich gelöster Fremdstoff im Reaktionsbereich in der Matrix zunächst gebunden wird und der in der Matrix ursprünglich gelöste Fremdstoff verzögert so freigesetzt wird, dass der ursprünglich gelöste Fremdstoff und etwaiger ursprünglich gebundener Fremdstoff im gleichen Verfahrensschritt gemeinsam aus der Matrix freigesetzt werden. Dies erfolgt insbesondere bei der Bestimmung des Gesamtgehalts an SO₂, das in der Matrix in gelöster und gebundener Form vorliegt.

Vorteilhaft kann die Matrix zum Abbinden des gelösten Anteils an Fremdstoff mit einem Adduktbildner vermischt werden. Nach Abbinden des ursprünglich gelösten Fremdstoffs kann ein Reagenz zugegeben werden, welches den ursprünglich gelösten Fremdstoff und etwaigen ursprünglich gebundenen Fremdstoff aus der Matrix austreibt. Bevorzugt kann dabei der ausgetriebene Fremdstoff durch ein Trägergas einem Sensor zur Detektion des Fremdstoffs zugeführt werden.

Alternativ ist auch denkbar, statt der Zugabe eines Adduktbildners die Matrix abzudecken, um einen frühzeitig freigesetzten Fremdstoff zurückzuhalten und erst freizugeben, wenn auch der ursprünglich in der Matrix gebundene Anteil des Fremdstoffs freigesetzt wird. Denkbar ist auch, die Zugabe eines Adduktbildners und das Abdecken der Matrix zu kombinieren.

Aufgrund der nicht im chemischen Gleichgewicht durchgeführten Messung, z.B. des SO0₂-Gehalts im Wein, sind unterschiedliche zeitabhängige Konzentrationsprofile für unterschiedlich stark gebundene sulfithaltiger Spezies zu erwarten. So lässt sich feststellen, dass in der Wein-Probe als freies SO₂ vorliegendes Sulfit einen zeitlich nach vorne hin verlagerten Peak in der Messkurve der Konzentration ergibt, der nach dem Erreichen des Konzentrationsmaximums relativ steil abnimmt. Für Spezies, in denen das chemische Gleichgewicht sehr stark auf der Seite des gebundenen SO₂ liegt, lassen sich Kurven feststellen, bei denen der Peak in dem zeitabhängigen Konzentrationsprofil zeitlich stark nach hinten verlagert ist und dessen Abnahme nach dem Konzentrationsmaximum relativ langsam erfolgt. Gemäß der Erfindung kann durch die verzögerte Freisetzung des ursprünglich ungebundenen Fremdstoffs aus der mit dem Adduktbildner abgebundenen Form auch bei Vorliegen unterschiedlichster Spezies in nicht bekanten Konzentrationsverhältnissen eine Angleichung des Konzentrationsprofils zur einfachen mathematischen Konzentrationsbestimmung über die Summe aller Spezies des jeweiligen Fremdstoffs, z.B. der Sulfit-Spezies in Wein, Trockenobst, Fruchtsäften, Bier und dergleichen, erreicht werden.

Beim Nachweis des gesamten Fremdstoffgehalts in der Matrix, der ursprünglich gelöst und gebunden vorliegt, kann das Fremdstoff-Addukt durch Wärme und Säureeinfluss wieder in die Edukte (Adduktbildner und Fremdstoff) überführt werden. Mittels eines Gasstroms wird der gelöste Fremdstoff, z.B. das gelöste SO₂, aus der Lösung in den Gasstrom überführt und über einen fremdstoffempfindlichen Sensor geleitet, in welchem ein konzentrationsabhängiges Signal erzeugt wird.

Die quantitative Freisetzung des gebundenen Fremdstoffs, z.B. des gebundenen SO₂ in einer Matrix, z.B. in Wein, erfordert drastische Umgebungsbedingungen (hohe Temperatur, starke Säure) oder aber eine zeitlich gesehen lange Einstellung des chemischen Gleichgewichts. Da die Messung erfindungsgemäß durch einen Gasstrom erfolgt, durch die Probe geleitet wird, kann die quantitative Umsetzung schnellstmöglich erfolgen.

Das Erhitzen der Probe im Probenbehälter kann bevorzugt durch die anfallende HydratationsenergieNerdünnungswärme erfolgen, die frei wird, wenn konzentrierte Säure mit einem wässrigen Medium in Berührung kommt. Vorteilhaft ist hier der Wegfall mechanischer und/oder elektrischer Komponenten, sowie die Möglichkeit einer gemeinsamen Leitungsführung für Reagenzien zum Nachweis gelösten Fremdstoffs (z.B. freies SO₂ bei niedrigen Temperaturen ("kalt") und des gesamten SO₂, d.h. des ursprünglich gelösten und gebundenen SO₂ bei erhöhten Temperaturen ("warm")).

Die Bestimmung des frei vorliegenden SO₂ (in der Matrix gelöstes SO₂) erfolgt bevorzugt unter möglichst milden Bedingungen, so dass das gebundene SO₂ durch die Reagenzien nicht freigesetzt wird, da dies die Messung verfälschen würde. Das Referenzverfahren sieht eine Kühlung des Reagenz/Matrixgemisches auf bevorzugt weniger als 20°C, besonders bevorzugt auf ca. 10°C vor.

Vorzugsweise erfolgt bei der Bestimmung des Gesamtgehalts an gelöstem und gebundenem SO₂ ein Abbinden des SO₂ durch Zugabe eines Adduktbildners im Überschuss. Vorteilhaft kann als Adduktbildner Brenztraubensäure bzw. ein Salz davon, eingesetzt werden. Das Salz hat den Vorteil, dass es in festem Zustand vorliegt und in dieser Form hinreichend chemisch stabil ist. Die Brenztraubensäure weist eine relativ hohe Bindungsaffinität auf und liefert stabile Bisulfit-Addukte. Eingesetzt wird diese vorzugsweise in Form einer Lösung. Diese Lösung liegt in dem Probenbehälter vordosiert vor. Sie kann jedoch auch durch den Anwender mittels einer Tropfpipette zugegeben werden.

Zur besseren Durchmischung der Probe bei Zuführen des Säure-Reagenzes wird vorzugsweise ein Probenbehälter verwendet, auf dessen Boden sich z.B. als Substrat eine Agar-Schicht, eventuell versetzt mit etwas Brenztraubensäure oder einem Salz davon, befindet. Diese Schicht verflüssigt sich bei Säure/Wärmeeinftuss auf und sorgt für eine Durchmischung der Lösung (z.B. Wein als Matrix). Auf diese Agar-Schicht wird die Brenztraubensäure bzw. das Salz der Brenztraubensäure als Lösung oder auch in fester Form gegeben und schließlich mit der Probe versetzt. Wird als Reagenz Schwefelsäure H₂SO₄ zugegeben, so würde diese aufgrund ihrer hohen Dichte nach unten sinken, während die Probe, z.B. Wein, wegen ihrer geringeren Dichte oben aufschwimmen würde. Durch den Lösevorgang des Substrats erfolgt jedoch eine vorteilhafte Durchmischung, so dass Probe und Säure hinreichend in Kontakt kommen, auch bei kleinen Probenvolumina.

Vorgeschlagen wird zudem ein Verfahren zur Bestimmung eines Gehalts wenigstens eines Fremdstoffs in einer Matrix eines festen oder flüssigen Lebensmittels, welches jedoch nicht Teil der Erfindung ist, bei dem ein ursprünglich gelöster Fremdstoff im Reaktionsbereich in der Matrix zunächst gebunden wird, und in der Matrix ursprünglich gelöste Fremdstoff verzögert so freigesetzt wird, dass der ursprünglich gelöste Fremdstoff und etwaiger ursprünglich gebundener Fremdstoff im gleichen Verfahrensschritt gemeinsam aus der Matrix freigesetzt werden; und die Bestimmung des Gehalts des im gleichen Verfahrensschritt freigesetzten Fremdstoffs mittels eines Sensors erfolgt.

Vorteilhaft kann zur Verzögerung der Freisetzung des Fremdstoffs ein Adduktbildner zugegeben werden, der gelösten Fremdstoff bindet. Bevorzugt kann zur Freisetzung des Fremdstoffs ein Reagenz zugesetzt werden.

Weiterhin kann vorteilhaft der Adduktbildner Brenztraubensäure oder ein Salz der Brenztraubensäure und das Reagenz Schwefelsäure und /oder Phosphorsäure aufweisen.

Bevorzugt kann die Matrix ein flüssiges und/ oder ein festes Lebensmittel, insbesondere Wein oder ein Weinbestandteil, Fruchtsäfte, Bier, Trockenobst, insbesondere ein Auszug aus Trockenobst sein und der Fremdstoff im Lebensmittel gebundenes und/oder gelöstes SO₂ sein.

Ebenfalls nicht Teil der Erfindung ist die Verwendung eines oder mehrerer Reagenzien und/oder eines oder mehrerer Adduktbildner, die geeignet sind zur Durchführung eines Verfahrens zum Testen von festen und flüssigen Lebensmitteln, insbesondere Wein, Fruchtsäfte, Bier, Trockenobst und dergleichen auf einen oder mehrere Fremdstoffgehalte.

Anhand von in der Zeichnung dargestellten Ausführungsbeispielen wird die Erfindung nachfolgend näher beschrieben. Es zeigen in schematischer Darstellung
- Fig. 1: einen bevorzugten Messaufbau einer Vorrichtung nach der Erfindung;
- Fig. 2: eine perspektivische Ansicht einer bevorzugten Vorrichtung nach der Erfindung;
- Fig. 3: als Detail herausgehoben einen in einen Reagenzienbereich der Vorrichtung der Fig. 2 eingesetzten Reagenzienbehälter;
- Fig. 4: als Detail einen an einen Reaktionsbereich angeschlossenen Probenbehälter;
- Fig. 5a, 5b: eine bevorzugter Sensor (Fig. 5a) und eine Referenzelektrode (Fig. 5b) für den Sensor;
- Fig. 6a-6c: einen bevorzugten konfektionierten Probenbehälter in verschiedenen Stadien der Vorbereitung eines Nachweises; und
- Fig. 7: ein Flussdiagramm eines bevorzugten Messablaufs.

Gleiche oder gleich wirkende Elemente werden in den Zeichnungen mit gleichen Bezugszeichen beziffert.

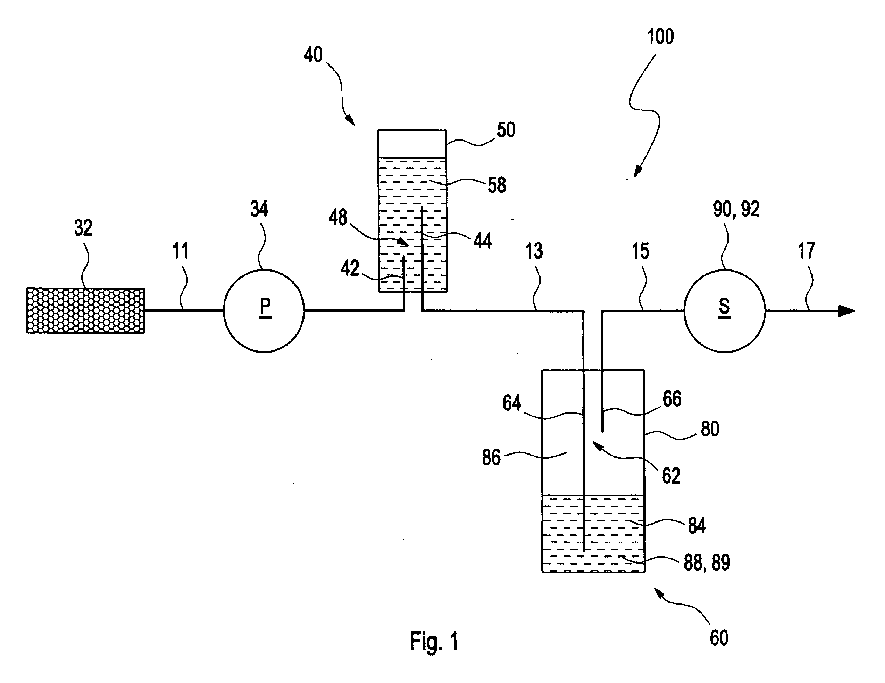

Fig. 1 zeigt zur Erläuterung der Erfindung schematisch den Aufbau einer bevorzugten Vorrichtung 100. Ein Reagenzienbehälter 50 ist über eine Überführungsleitung 13 in Strömungsverbindung mit einem Probenbehälter 80 verbunden. Stromab des Probenbehälters 80 ist ein Sensorbereich 90 vorgesehen, die mit dem Probenbehälter 80 mit einer Leitung 15 verbunden ist. Eine Ausgangsleitung 17 führt von dem Sensorbereich 90 nach außen. Eine Pumpe 34 fördert ein Trägermedium über die Pumpleitung 11 zum Reagenzeinbereich 40. Das Trägermedium ist vorzugsweise ein Gas, z.B. Umgebungsluft, die zweckmäßigerweise über einen Filter 32 angesaugt wird. Das Trägermedium kann auch gegebenenfalls aus einer Gasflasche entnommen werden. Die Pumpe 34 kann stromauf des Reagenzienbehälters 50 in der Pumpleitung 11 oder auch stromab des Reagenzienbehälters 50, z.B. in der Überführungsleitung 13, angeordnet sein.

Dem Probenbehälter 80 ist gezielt ein Reagenz 58, z.B. Schwefelsäure (H₂SO₄) aus dem Reagenzienbehälter 50 zuführbar, das den im Probenbehälter 80 in einer Matrix 88 enthaltenen ungebundenen und gegebenenfalls gebundenen Fremdstoff 89 komplett freisetzt. Dabei ist vorgesehen, dass ein gegebenenfalls ungebundener (gelöster) Fremdstoff vorher vollständig abgebunden wird, insbesondere durch Zusatz eines Adduktbildners.

Der Reagenzienbehälter 50 ist auf ein Nadelsystem 48 aufgesteckt, das z.B. zwei Hohlnadeln 42 und 44 umfasst. Die Hohlnadeln 42, 44 des Nadelsystems 48 ragen vorzugsweise nach oben und stehen parallel zueinander. Eine Ausgestaltung z.B. mit koaxialen Hohlnadeln oder Nadeln mit innenliegendem Schlauch, ist ebenso möglich. In einer nicht dargestellten günstigen Weiterbildung können die Hohlnadeln 42, 44 als Bauteil in einen Trägerkörper integriert sein, der Anschlüsse zur Pumpleitung 11 und zur Überführungsleitung 13 aufweist. Das Nadelsystem 48 kann dann einfach ausgetauscht werden.

Über die eingangsseitige Hohlnadel 42 tritt das Trägermedium in den Reagenzienbehälter 50 ein. Über die ausgangsseitige Hohlnadel 44 wird das Reagenz aus dem Reagenzienbehälter 50 entnommen und dem Probenbehälter 80 zugeführt. Dort wird eine Matrix 88, die den nachzuweisenden Fremdstoff 89 enthält, z.B. Wein mit einem Gehalt an SO₂, mit dem Reagenz 58 versetzt und der Gehalt an Fremdstoff 89 in dem Sensorbereich 90 bestimmt. Zum Nachweis ist im Sensorbereich 90 ein Sensor 92 vorgesehen. Der Fremdstoff 89 wird vorzugsweise gasförmig in den Sensorbereich 90 und zum Sensor 92 geleitet. Gegebenenfalls kann der Fremdstoff 89 dem Sensor 92 auch in einer Lösung zugeführt werden.

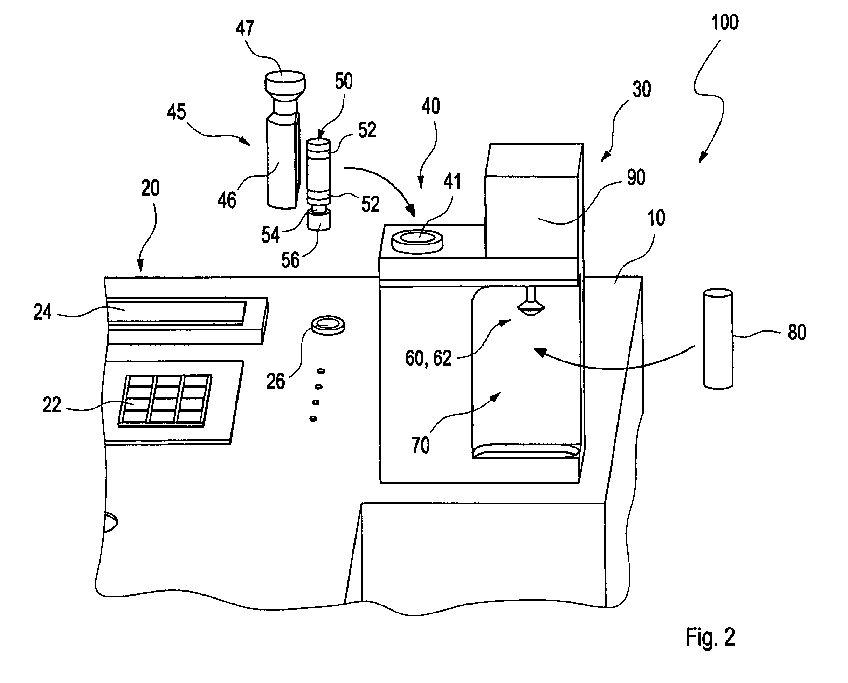

Bevorzugt ist der Reagenzienbehälter 50 als vorbefüllte, insbesondere vordosierte Ampulle ausgebildet, welche der Anwender im Bedarfsfall einfach in die später detaillierter beschriebene Vorrichtung 100 einsetzt. Der als vordosierte Ampulle ausgebildete Reagenzienbehälter 50 enthält ein oder mehrere für den spezifischen Nachweis eines Fremdstoffs geeignete und/oder notwendige Reagenzien 58 in genau der Dosierung, die auf die im Probenbehälter 80 vorgesehene Menge der Probe (Matrix 88 mit Fremdstoff 89) abgestimmt ist. Der Reagenzienbehälter 50 ist so konfektioniert, d.h. in seinen Abmessungen so ausgebildet, dass er in einen entsprechend komplementär ausgebildeten Reagenzienbereich 40 (Fig. 2) der Vorrichtung 100 eingeführt werden kann. Eine Fehlbedienung der Vorrichtung 100 kann dadurch vermieden werden.

Der Reagenzienbehälter 50 kann beispielsweise in der Form einer Durchstech-Ampulle mit einem Durchstich-Septum als Verschluss mit einem Zwei-Nadelsystem 48 der Vorrichtung 100 verbunden werden. Der Reagenzienbehälter 50 kann auch eine Ampulle oder dergleichen sein.

Dabei wird der Reagenzienbehälter 50 mit seiner Unterseite auf das Nadelsystem 48 aufgesteckt. Durch die "Kopfüber"-Montage kann der Reagenzienbehälter 50 zuverlässig entleert werden. Denkbar ist jedoch alternativ auch eine Montage, bei der der Reagenzienbehälter 50 mit dem Verschluss nach oben oder zur Seite an den Reagenzienbereich 40 angeschlossen wird.

Das von der Pumpe 34 geförderte Trägermedium, z.B. Luft, das später zur Messung benötigt wird, tritt durch die im Bild links angeordnete eingangsseitige Hohlnadel 42 in den als Ampulle ausgebildeten Reagenzienbehälter 50 ein. Durch den Überdruck wird das Reagenz 58 durch die ausgangsseitige rechte Hohlnadel 44 in das weitere Mess-System der Vorrichtung 100 überführt. Mit dem von der Pumpe 34 gelieferten nachfolgenden Trägermedienstrom wird der mit dem Reagenz 58 aus der Matrix 88 ausgetriebene Fremdstoff 89, SO₂, schließlich aus dem Probenbehälter 80 zum Sensor 92 des Sensorbereichs 90 geführt. Vorzugsweise taucht die eingangsseitige Hohlnadel 42 weniger tief in den Reagenzienbehälter 50 ein als die ausgangsseitige Hohlnadel 44, um einen gegebenenfalls vorhandenen oder beim Einstich erzeugten Überdruck im Reagenzienbehälter 50 durch die ausgangsseitige Hohlnadel 44 abzubauen, bevor die eingangsseitige Hohlnadel 42 mit dem Inneren des Reagenzienbehälters 50 in Kontakt kommt.

Vorzugsweise liegt das eingeschlossene Volumen des Reagenzienbehälters 50 zwischen 0,2 ml und 15 ml, vorzugsweise zwischen 0,5 ml und 10 ml. Das eingeschlossene Volumen des Reagenzienbehälters 50 kann ganz oder teilweise mit dem Reagenz 58, z.B. einer Flüssigkeit, befüllt sein. Vorzugsweise ist der Reagenzienbehälter 50 dafür ausgebildet, eine Reagenzienmenge für genau eine Messung aufzunehmen. Für jede Messung kann also ein neuer Reagenzienbehälter 50 eingesetzt werden. Vorzugsweise sind die Füllmengen von Reagenzienbehälter 50 und Probenbehälter 80 genau aufeinander abgestimmt. Mit besonderem Vorteil kann der Probenbehälter 80 bereits eine oder mehrere spezifische Chemikalien in genau dosierter Menge für den Nachweis des Fremdstoffs 89 enthalten. Vorzugsweise kann der Reagenzienbehälter 50 und der Probenbehälter 80 als Nachweis-Analytiksatz (Set) bereitgestellt werden. Der Anwender muss nur noch den Probenbehälter 80 mit einer vorbestimmten Menge der Matrix 88, die den Fremdstoff 89 enthält, versetzen. Der Anwender kann daher immer unter genau definierten Bedingungen die Bestimmung des Fremdstoffgehalts in der Matrix 88 durchführen.

Selbst in Fällen, bei denen das Reagenz 58 korrosives Verhalten aufweist und/oder empfindlich gegen Luft, Feuchtigkeit und/oder sonstige Inhaltsstoffe der umgebenden Raumluft am Einsatzort beim Nachweis oder beim Transport und der Lagerung sein sollte, ist eine stabile Messung und ein sicherer Überführung in den Probenbehälter 80 möglich. Außerhalb der Vorrichtung 100 ist das Reagenz 58 im Reagenzienbehälter 50 bis zur Verwendung dicht gekapselt und wird erst im Bedarfsfall nach dem Aufstecken des Reagenzienbehälters 50 auf das Nadelsystem 48 zugänglich. Zudem ist die Menge des Reagenzes 58 auf einen für den Nachweis notwendige Menge beschränkt und braucht nicht vom Anwender dosiert zu werden.

Bei einer bevorzugten Verwendung der Erfindung wird SO₂ als Fremdstoff 89 in Wein als Matrix 88 nachgewiesen. Zum Nachweis des Gesamtgehalts an gelöstem und gebundenem Fremdstoff, insbesondere SO₂ in Wein wird als Reagenz 58 vorzugsweise H₂SO₄ mit einer Konzentration so eingesetzt, dass nach der Mischung von dem Reagenz 58 mit der Probe (incl. Zusätze wie Adduktbildner etc.) eine Säurekonzentration von 15%-85%, vorzugsweise 40%-85% vorliegt. Es können auch andere organische oder anorganische Säuren, z.B. Phosphorsäure, günstigerweise in vergleichbaren Konzentrationsbereichen eingesetzt werden.

Vorzugsweise sind das Nadelsystem 48 wie auch die darauf stromabwärts folgenden Leitung 13 gegen Korrosion geschützt. Günstigerweise können für das Nadelsystem 48 und/oder die Leitung 13 und für die anderen Leitungen 11, 15 und 17 Edelstahlkapillaren aus handelsüblichen Edelstahlsorten eingesetzt werden, z.B. Edelstähle 1.4401, 1.4571, 1.4541, 1.4301, 1.4404 oder Legierungen, insbesondere Legierungen, die Cr, Mo, Ni enthalten. Denkbar sind auch andere Materialien wie Edelmetalle, Glas, Keramik, Kunststoff.

Edelstahl kann üblicherweise durch das eingesetzte Reagenz 58, das beispielsweise halbkonzentrierte Schwefelsäure enthält, bei längerer Einwirkdauer stark angegriffen werden. Gemäß einer vorteilhaften Weiterbildung der Erfindung wird das Reagenz 58 daher mit einem Inhibitor versetzt, der die Korrosion vermindert oder ganz verhindert. Zum Nachweis von SO₂ wird z.B. eine 40%-85%ige Schwefelsäure durch Zusatz eines oder mehrerer Inhibitoren, z.B. Eisen(III)-Salze, derart modifiziert, dass ein Angriff auf die Edelstahlkapillare nicht mehr erfolgt. Zweckmäßigerweise wird der Inhibitor dem Reagenz 58 zugesetzt und kann zweckmäßigerweise bereits im vorbefüllten Reagenzienbehälter 50 enthalten sein.

Wird in der erfindungsgemäßen Vorrichtung 100 mit unterschiedlichen Reagenzien 58 gearbeitet, etwa um mit verschiedenen Methoden den Fremdstoff 89 in der Matrix 88 nachzuweisen, werden zweckmäßigerweise alle entsprechenden Reagenzien 58, die in einem Reagenzienbehälter 50 eingesetzt werden, mit einem geeigneten Inhibitor versetzt, um eine definierte Inhibitorkonzentration aufrecht zu erhalten. Ein solcher Fall kann z.B. auftreten, wenn in der Vorrichtung 100 mit zwei verschiedenen Methoden "freier" Fremdstoff 89, d.h. solcher, der in Matrix 88 nicht gebunden vorliegt, sondern in Lösung ist, und der gesamte Gehalt an Fremdstoff 89, nämlich freier Fremdstoff und gebundener Fremdstoff, in der Matrix 88 nachgewiesen werden sollen. Für die Bestimmung des freien und des gesamten Fremdstoffgehalts werden z.B. zwei unterschiedliche Reagenzien 58 im gleichen System eingesetzt, die z.B. beide mit einer geeigneten Menge Inhibitor, z.B. Eisen (III)-Salzen, versetzt sind.

Wird die Inhibitorkonzentration gegenüber dem Reagenz 58 deutlich verringert, z.B. durch nicht ausreichendes Nachspülen der Kapillare mit reinem Wasser, können Korrosionserscheinungen auftreten. Daher ist ein ausreichendes Nachspülen der Kapillare mit reinem Wasser oder anderen geeigneten Spüllösungen empfehlenswert. Vorteilhaft ist, ein Nachspülen mit einer wässrigen Lösung des Inhibitors durchzuführen. Dies kann zusätzlich oder alternativ dazu erfolgen, dass das Reagenz 58 mit dem Inhibitor gemischt wird.

Nach dem Anschließen des Reagenzienbehälters 50 kann das Reagenz 58 durch das durch die Pumpe 34 geförderte Trägermedium aus dem Reagenzienbehälter 50 vollständig in den Probenbehälter 80 überführt werden.

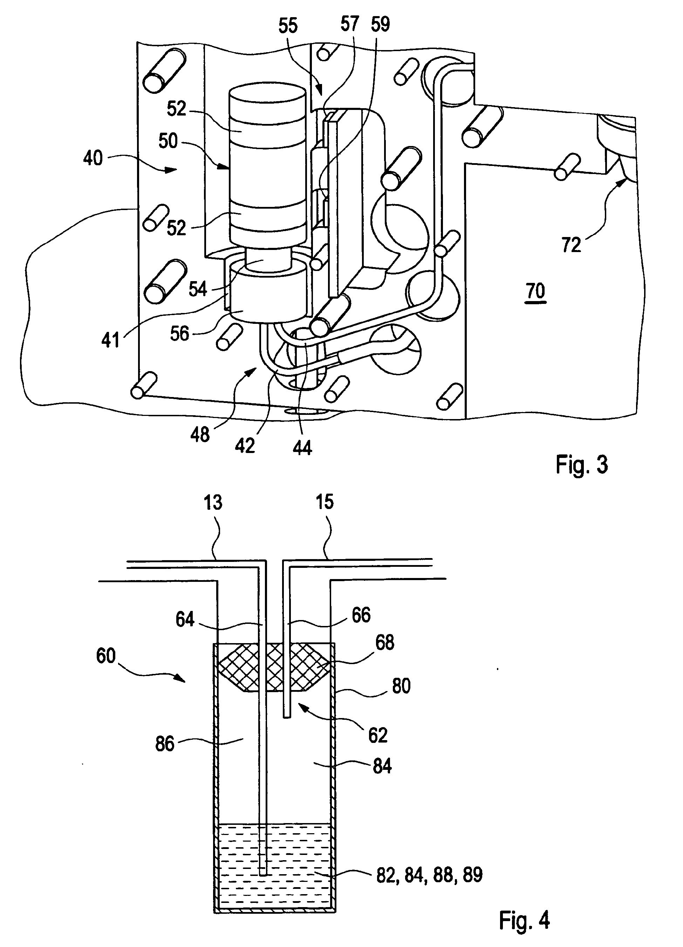

Der Probenbehälter 80 ist vorzugsweise konfektioniert, so dass er an einen geeignet ausgebildeten Reaktionsbereich 60 der Vorrichtung 100 anschließbar ist. Dazu kann der Reaktionsbereich 60 eine konusförmige Kupplung 72 aufweisen, die außenumfänglich einen Dichtring aufweisen kann, über den der beispielsweise zylinderförmige Probenbehälter 80 geschoben wird. Statt einem Dichtring kann auch eine Presspassung oder ein anderes geeignetes Dichtmittel vorgesehen sein. Die Kupplung 72 ist in der Detaildarstellung der Fig. 3 in der Vertiefung 70 zu erkennen.

Der Probenbehälter 80 weist vorzugsweise Abmessungen auf, die es erlauben, mit dessen Füllung genau eine Messung zum Nachweis des Fremdstoffs 89 in einer Matrix 88 mit abgestimmten Volumina von Matrix 88 und einem Adduktbildner 84 durchzuführen. Der Zusatz des Adduktbildners 84 erlaubt die gleichzeitige Bestimmung des Gehalts an gelöstem und gebundenen Fremdstoffs in form von Sulfit bzw. SO₂. Der Adduktbildner 84 ist vorzugsweise in dem vorbefüllten Probenbehälter 80 enthalten und braucht nicht vom Anwender zugeführt werden. Alternativ kann der Adduktbildner 84 separat in geeigneter Dosierung im bevorzugten Nachweisanalyse-Satz dem Anwender bereitgestellt und erst bei der Messung zugeführt werden.

Der Reaktionsbereich 60 umfasst vorzugsweise ein Anschlusssystem 62 mit einem ersten eingangsseitigen Anschluss 64, über die das Reagenz 58 zuführbar ist, und einem zweiten ausgangsseitigen Anschluss 66, über den der Fremdstoff 89 zum Sensorbereich 90 leitbar ist. Die Anschlüsse 64 und 66 ragen aus der konusförmigen Kupplung heraus, wobei wenigstens einer der Anschlüsse 64 durch einen Stopfen 68 (Fig. 4) führt, der als Spritzschutz für den Probenbehälter 80 dient. Die Anschlüsse 64, 66 können als Hohlnadeln ausgebildet sein oder auch als Schläuche oder Röhrchen ausgebildet sein. Eine koaxiale Ausgestaltung mit ineinandersteckenden Hohlnadeln und/oder Schläuchen und/oder Röhrchen ist ebenso möglich.

Ist der Probenbehälter 80 mit seiner nach oben offenen Öffnung auf die Kupplung des Reaktionsbereichs 60 aufgesteckt, ragt der eingangsseitige Anschluss 64, welcher das Reagenz 58 zuführt, vorzugsweise tiefer in den Probenbehälter 80 hinein als der ausgangsseitige Anschluss 66, über welchen der nachzuweisenden Fremdstoff 89 bzw. ein Reaktionsprodukt desselben aus dem Probenbehälter 80 zum Sensorbereich 90 geführt wird. Der Anschluss 64 ragt insbesondere in einen Mischbereich hinein, in dem Matrix 88, Fremdstoff 89 und, im Falle des Nachweises des Gesamtgehalts an SO₂, der Adduktbildner 84 vermischt sind, so dass das Reagenz 58 aus dem Anschluss 64 möglichst innig mit dem in der Matrix 88 gebunden und/oder frei enthaltenen Fremdstoff 89 in Kontakt kommen kann. Der Fremdstoff wird durch Einwirken des Reagenz 58 in den Gasraum 86 über dem Mischbereich freigesetzt und kann durch den ausgangsseitigen Anschluss 66, der in den Gasraum 86 ragt, aus dem Probenbehälter 60 Richtung Sensorbereich 90 austreten.

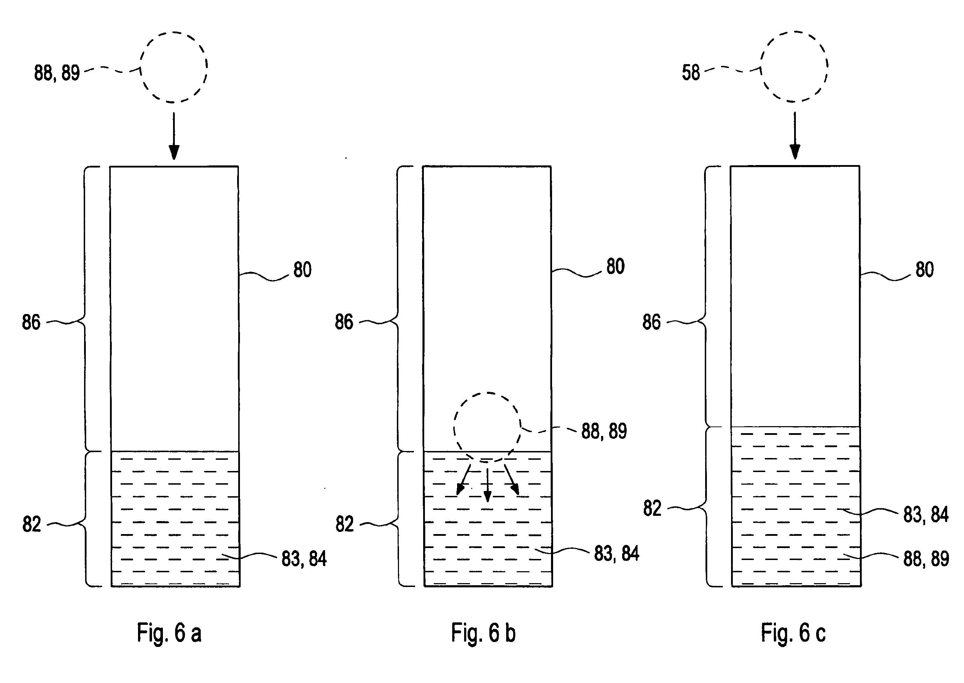

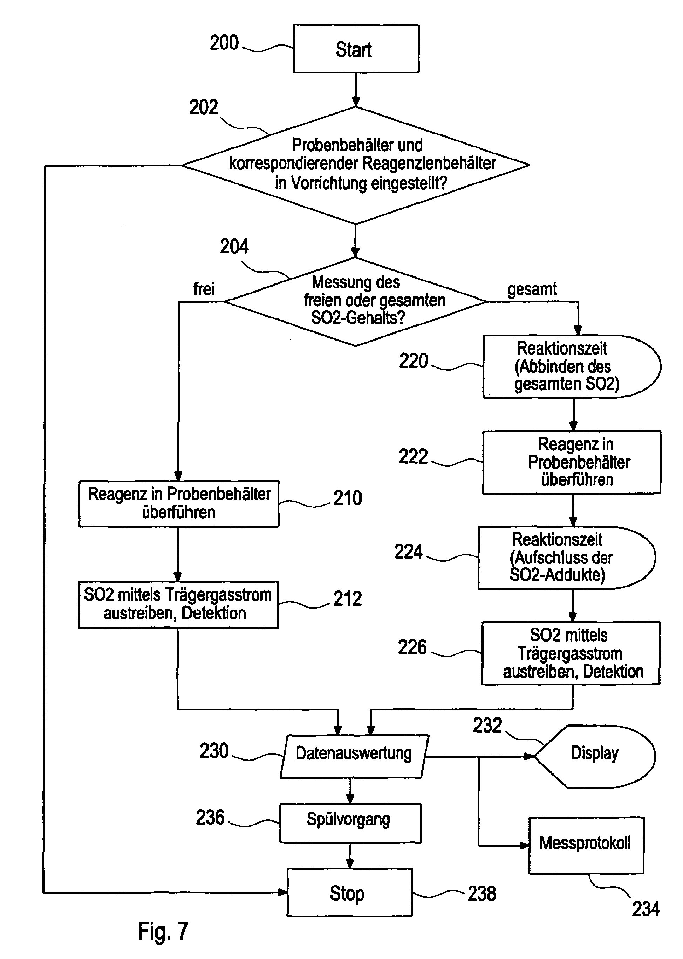

Beim Nachweis des Fremdstoffs 89 kann es günstig sein, den Probenbehälter 80 auf einer gewünschten Temperatur oder in einem gewünschten Temperaturbereich zu halten. Der Probenbehälter 80 kann gegebenenfalls geheizt werden oder auch gekühlt werden, je nach aktuell ausgeführter Reaktion im Probenbehälter 80. Weitere Details des Ablaufs des Nachweises sind in den Fig. 6 und 7 beschrieben.

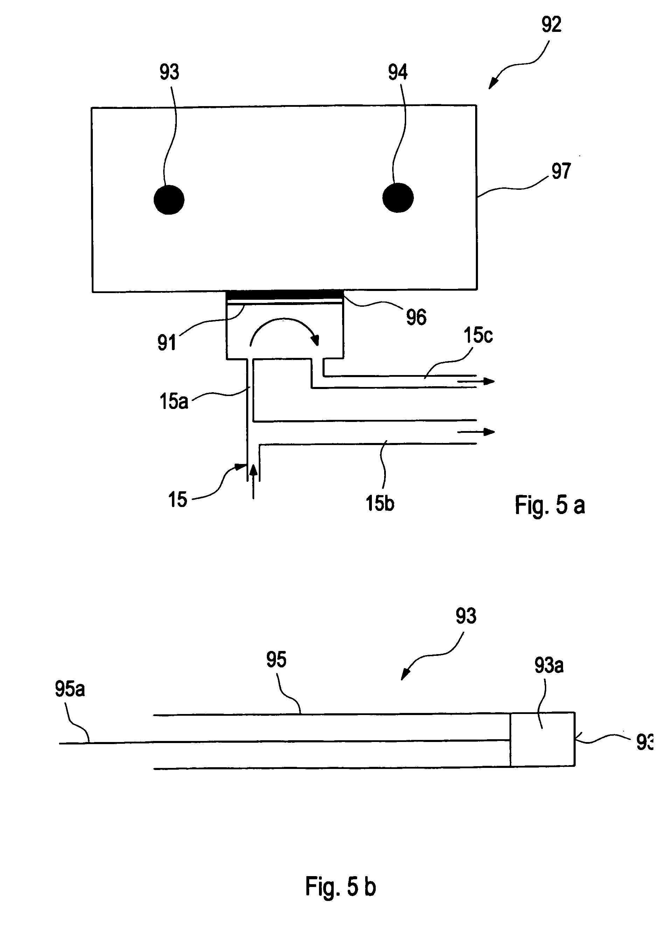

Der aus der Matrix 88 durch das Reagenz 58 freigesetzte Fremdstoff 89 gelangt über den Anschluss 66 zum Sensorbereich 90. Vorzugsweise weist der Sensorbereich 90 einen elektrochemischen Sensor 92 auf, der im Detail in den Fig. 5a und 5b erläutert ist.

Fig. 2 zeigt beispielhaft eine mögliche Ausführung einer bevorzugten Vorrichtung 100 zum Nachweis eines Fremdstoffs in einer Matrix entsprechend dem in Fig. 1 dargestellten prinzipiellen Aufbau. Zur Vermeidung unnötiger Wiederholungen wird auf die dort beschriebenen Komponenten verwiesen, soweit diese hier nicht weiter erläutert sind.

Die Vorrichtung 100 ist in einem Gehäuse 10 angeordnet, die einen Kontrollteil 20, einen Reagenzienbereich 40 mit einer als Vertiefung ausgebildeten Aufnahme 41 für den Reagenzienbehälter 50 und einen Analysenteil 30 mit den Reaktionsbereich 60 für den Probenbehälter 80 und den Sensorbereich 90 umfasst. Eine Leitungssystem umfassend eine Pumpleitung 11 und eine Überführungsleitung 13 (Fig. 1) dient der Überführung des Reagenz 58 (Fig. 1) aus dem Reagenzienbehälter 50 in den Probenbehälter 80. Es kann auch eine separate Pumpe bzw. ein Druckvorratsgefäß den Trägermedienstrom herstellen und die Dosierung als separate Einheit bereitgestellt werden.

Der Kontrollteil 20 umfasst z.B. eine Tastatur 22, eine Anzeige 24, einen Startknopf 26 und eine entsprechende Recheneinheit (nicht dargestellt) zur Steuerung und gegebenenfalls Auswertung der Messung und zur Überwachung der Vorrichtung 100.

Die Vorrichtung 100 ist sehr kompakt und erlaubt eine sichere Handhabung von Reagenzienbehälter 50 und Probenbehälter 80 mit genau aufeinander abgestimmten Mengen und Volumina der eingesetzten chemischen Komponenten. Der Reagenzienbehälter 50 kann verwechslungssicher eingesetzt werden, wobei die Vorrichtung 100 im Reagenzienbereich 40 eine Sensoreinrichtung 55 aufweist, z.B. eine Lichtschranke 57, 59, einen mechanischen Schalter oder dergleichen, die das Vorhandensein des Reagenzienbehälters 50 feststellen kann. Der Reagenzienbehälter 50 kann dazu z.B. reflektierende Markierungen 52 aufweisen, anhand derer die Vorrichtung den eingesetzten Reagenzienbehälter 50 erkennen kann. Ferner kann dies als eine einfache Codierung eine Erkennung von Reagenzienbehältern 50 unterschiedlichen Reagenzien ermöglichen. Es können jedoch auch andere Codierungen, z.B. Barcode, mechanische Codierung etc., dazu verwendet werden, so dass die Vorrichtung 100 die Ausführung einer Messung mit nicht füreinander vorgesehenen Reagenzien und Proben sicher unterbinden kann. Die Betriebssicherheit ist dadurch weiter, auch bei ungeübten Anwendern, erhöht.

Die Aufnahme 41 im Reagenzienbereich 40 ist als Vertiefung ausgebildet, in welche der Reagenzienbehälter 50 eintauchen kann. Dazu wird der Reagenzeinbehälter 50 in einen Einsatz 45 eingesetzt. Vorzugsweise kann der Reagenzienbehälter 50 ein Volumen zwischen 0,2 ml und 15 ml, vorzugsweise zwischen 0,5 ml und 10 ml, des Reagenz 58 aufnehmen.

Eine bevorzugte Ausgestaltung des Reagenzienbehälters 50 sieht vor, dass dieser einen zylindrischen Querschnitt aufweist mit einer Einschnürung 54 an einem Ende, an die ein Verschluss 56, beispielsweise einem Durchstich-Septum, anschließt. Der Einsatz 45 umschließt den Reagenzienbehälter 50 mit seinem Körper 46 teilweise, wobei ein Bereich für die Erkennung der Markierungen 52 frei bleibt, und greift in die Einschnürung 54 ein, so dass der Reagenzienbehälter 50 mit dem Träger 45 in die als Vertiefung ausgebildete Aufnahme 41 des Reagenzienbereichs 40 hineingedrückt werden kann, um das Nadelsystem 48 (Fig.1) in den Reagenzienbehälter 50 einzuführen. Am oberen Ende weist der Einsatz 45 einen Halteknopf 47 auf, der beim Einsetzen des Reagenzienbehälters 50 aus der Vertiefung 41 herausragt. Damit lässt sich der Reagenzienbehälter 50 nach der Messung leicht wieder aus der Vorrichtung 100 entfernen.

Die Aufnahmemimik 60 ist in einer Vertiefung 70 angeordnet, in die der Probenbehälter 80 eingesetzt und einen Konus des Reaktionsbereichs 60 aufgeschoben werden kann, über den das Anschlusssystem 62, z.B. ein Nadelsystem, zugänglich ist. In der Vertiefung 70 kann der Sensor angeordnet sein, z.B. eine Lichtschranke, ein Mikroschalter oder dergleichen, der ein Vorhandensein des Probenbehälters 80 erkennt und an die Steuerung der Vorrichtung 100 weiterleitet. Ist der Probenbehälter 80 nicht eingesetzt, kann die Messung nicht gestartet werden.

Fig. 3 zeigt den Reagenzienbereich 40 der Vorrichtung 100 aus Fig. 2 mit einem in die Vertiefung 41 eingesetzten Reagenzienbehälter 50 als Teilaufriss-Darstellung. Der Reagenzienbehälter 50 ist mit seinem Verschluss 56 nach unten auf das Nadelsystem 48 mit den Hohlnadeln 42 und 44 aufgesteckt, wie in Fig. 1 beschrieben wurde. Der Einsatz 45, der den Reagenzienbehälter 50 umschließt, ist der Übersichtlichkeit wegen nicht dargestellt. Ein Sensor 55, z.B. eine Lichtschranke mit zwei Sende- und Empfängereinheiten 57, 59, ist neben dem Reagenzienbehälter 50 angeordnet und detektiert die Anwesenheit des Reagenzienbehälters 50, z.B. über reflektierende Markierungen 52 auf dem Reagenzienbehälter 50. Ist der Reagenzienbehälter 50 nicht eingesetzt, kann die Messung nicht gestartet werden. In einer daneben liegenden Vertiefung 70 ist noch andeutungsweise eine konusförmige Kupplung 72 zu erkennen, auf welche der Probenbehälter aufgeschoben werden kann (nicht dargestellt).

Fig. 4 zeigt, wie der bevorzugte Probenbehälter 80 an den Reaktionsbereich 60 angeschlossen sein kann. Der Reaktionsbereich 60 umfasst ein beispielhaft als Nadelsystem ausgebildetes Zuführsystem 62 mit einem als Hohlnadel ausgebildeten eingangsseitigen Anschluss 64, mit dem das Reagenz 58 (Fig. 1) zugeführt wird und einem als Hohlnadel ausgebildeten ausgangsseitigen Anschluss 66, mit der das den Fremdstoff 89 enthaltende Trägergas aus dem Probenbehälter 80 abgeführt wird. Das Zuführsystem 62 durchstößt einen Stopfen 68, welcher als Spritzschutz dient. Der als Hohlnadel eingangsseitige Anschluss 64 ragt in einen Mischbereich 82 hinein, in dem Matrix 88, Fremdstoff 89 und ein Adduktbildner 84 vermischt sind, so dass das Reagenz 58 aus dem als Hohlnadel ausgebildeten Anschluss 64 möglichst innig mit dem in der Matrix 88 gebunden und/oder frei enthaltenen Fremdstoff 89 in Kontakt kommen. Der als Hohlnadel ausgebildete ausgangsseitige Anschluss 66 ragt in den Gasraum 86 oberhalb des Mischbereichs 82 hinein. Der Anschluss 66 kann unterhalb oder oberhalb des Stopfens 68 enden, da dieser den Probenbehälter 80 nicht gasdicht abschließt.

In einer alternativen Anordnung kann der Sensor 92 in diesem Gasraum 86 angeordnet sein, statt in einem entfernt angeordneten Sensorbereich 90. Denkbar wäre auch ein Tauchsensor mit einer gaspermeablen Membran, der direkt in den Mischbereich 82 eintaucht.

Fig. 5a zeigt schematisch als bevorzugten elektrochemischen Sensor 92 ein Dreielektrodensystem mit einer Referenzelektrode 93, die in Fig. 5b detailliert dargestellt ist. Die Referenzelektrode 93 dient als Potentialbezugspunkt für die elektrochemische Messung, die sowohl potentiostatisch als auch galvanostatisch durchgeführt werden kann.

Der Sensor 92 sollte vorzugsweise mit einer chloridfreien Referenzelektrode 93 betrieben werden. Die Möglichkeiten sind in einer derartigen Anwendung, die mit wenig Aufwand und für relativ ungeübte Anwender verfügbar sein soll, stark eingeschränkt. Eine vorteilhaft einfache und umwelttechnisch günstige Lösung ist eine Blei/Bleisulfat-Elektrode (Pb/PbSO₄), die jedoch in der Literatur als nur bedingt stabil und daher in der Praxis nicht einsatztauglich beschrieben ist.

Durch eine bevorzugte elektrochemische Behandlung lässt sich diese jedoch entgegen der gängigen Meinung ständig regenerieren, so dass ein für die Analysedauer ausreichend stabiles Potential erhalten werden kann. Vor jeder Analyse sowie in den Pausenzeiten wird die Referenzelektrode 93 daher vorzugsweise regeneriert und bietet danach für ca. 5 Minuten ein stabiles Potential. Dieser Zeitraum ist für einen Messvorgang völlig ausreichend. Der Wert gilt für eine so genannte Mikroelektrode. Durch Vergrößerung der Oberfläche lässt sich diese Zeit auch noch entsprechend verlängern.

Der Vorteil des regenerierbaren Systems, insbesondere des Pb/PbSO₄-Systems, ist in mehreren Aspekten zu sehen. Das System der Referenzelektrode 93 ist chloridfrei, die Langzeitstabilität des Systems liegt bei mehr als einem Jahr im eingebauten Zustand. Es ist eine einfache und unkritische Handhabung möglich, und die Referenzelektrode 93 ist stabil im sauren Milieu. Es findet kein "Ausbluten" des Systems durch Konzentrationsunterschiede statt, und es treten keine Störungen oder Vergiftungen der Arbeitselektrode 96 (SO₂-sensitiv) auf.

Direkt nach Herstellung weist die Referenzelektrode 93 das theoretisch berechnete Potential auf. Nach Einbau findet üblicherweise eine deutliche Drift statt, die zu einer Abweichung von mehreren 100 mV führen kann. Dadurch wäre diese Elektrode zunächst nicht brauchbar für analytische Systeme, bei denen die Abweichung nur einige mV betragen darf. Nach der bevorzugten elektrochemischen Behandlung kann die Referenzelektrode 93 jedoch vor jeder Messung in wenigen Sekunden auf exakt das Ausgangspotential regeneriert werden.

Eine bevorzugte elektrochemische Behandlung kann durch Anlegen einer elektrischen Spannung an die Referenzelektrode 93 erfolgen, die einen Stromfluss ermöglicht. Typischerweise liegt das Potential der Referenzelektrode beim Regeneriervorgang etwa -1,5 bis -3 Volt bezogen auf die Gegenelektrode. Die im elektrochemischen Sensor 92 vorhandene Gegenelektrode 94 wird dabei als Gegenpol verwendet.

Die Arbeitselektrode 96 des elektrochemischen Sensors 92 ist auf einer porösen Membran 91 aufgebracht und kommt mit einem vorbeiströmenden Trägergas, z.B. Luft, das mit dem Fremdstoff 89 gemischt ist, z.B. Luft mit SO₂ gemischt, über die Leitung 15 aus dem Probenbehälter 80 (Fig. 4) in Kontakt. Der Trägergasstrom ist durch Pfeile angedeutet und wird über die Leitung 15c von der Membran 91 weggeführt. Die Referenzelektrode 93 befindet sich mit der Hilfselektrode 94, z.B. einem Golddraht, innerhalb des Sensorgehäuses 97 in einer leitfähigen Flüssigkeit. Vorzugsweise enthält die Flüssigkeit Sulfat-Ionen (SO4²⁻), besonders bevorzugt besteht die Flüssigkeit aus H₂SO₄.

Um ein Austrocknen der Membran 91 zu vermeiden, wird ein Großteil des Trägergasstroms an der Membran 91 vorbei geleitet. Dazu spaltet sich die Leitung 15 in einen sehr schmalen Zweig 15a auf, der viel schmäler ist als die Leitung 15, und in einen breiten Zweig 15b auf, der viel breiter ist als der Zweig 15a. Die Zweige 15b und 15c münden einzeln in die Leitung 17, mit der der Trägergasstrom aus dem Sensor 92 austreten kann, können aber auch vorher zusammengeführt werden. Alternativ können diese auch einzeln nach außen geführt werden.

Neben einer möglichen Austrocknung der Membran 91, vorzugsweise eine Teflonmembran, und/oder der Arbeitselektrode 96, die als Schicht auf der Membran 91 abgelagert sein kann, kann durch die Aufspaltung des Trägergasstroms in die Zweige 15c und 15b auch ein zu großes Sensorsignal vermieden werden, ebenso können Einflüsse von Schwankungen im Gasfluss kompensiert werden. Diese Aufspaltung kann wahlweise auch schaltbar ausgeführt sein, so dass der gesamte Trägergasstrom mit dem Fremdstoff 89 über den Sensor 93 fließt. Alternativ kann die Aufspaltung auch durch eine weniger gasdurchlässige Sensor-Membran ersetzt werden.

Bei einem bevorzugten Spülgang zum Reinigen des Sensorbereichs 90 bzw. des Sensors 92 wird die Aufspaltung etwa 95% der Spülzeit ausgeschaltet, indem der Zweig 15b verschlossen wird, so dass eine erhöhte Spülwirkung erzielt werden kann.

Der Sensor 92 kann regelmäßig mit einem feuchten Medium gespült werden, um der Membran 91 ausreichend Feuchtigkeit zuzuführen.

Die Referenzelektrode 93 besteht vorzugsweise aus einem Pb/PbSO₄-System und kann, wie in Fig. 5b dargestellt ist, am Kopfende eines Röhrchens 95, z.B. eines Glasröhrchens, einen Pb-Einschluss 93a aufweisen, der mit einer Pb-Oberfläche 93b nach außen ragt. Der Pb-Einschluss 93a dichtet das Innere des Röhrchens 95 gegen eindringende Flüssigkeit ab. Der Bond-Draht 95a dient der elektrischen Kontaktierung des Pb-Einschlusses 93a bei der Regeneration der Referenzelektrode 93.

Ist die Referenzelektrode 93 aus einem Blei/Bleisulfat-System (Pb/PbₙXₘ-System) gebildet, kann die Referenzelektrode 93 durch eine elektrochemisch hervorgerufene Reaktion in seiner Beschaffenheit regeneriert werden. Bei Kontakt mit der leitfähigen Flüssigkeit bildet sich aus dem metallischen Pb das Sulfat PbS04 und bei der Regeneration aus PbSO4 wieder metallisches Pb. Vorzugsweise steht X für eine bestimmte Ionensorte in der leitfähigen Flüssigkeit. Insbesondere ist das Pb/PbₙXₘ-System ein Pb/PbSO₄-System mit n=1, m=1 und X=SO4²⁻. Die elektrochemisch aktiven Materialien bei einem Pb/PbSO₄-System sind Pb und Pb(II)-sulfat und/oder Pb und Pb(IV)-sulfat. Denkbar ist auch eine Referenzelektrode 93, bei der SO4²⁻ durch PbO ersetzt ist.

Grundsätzlich kann eine solche Referenzelektrode 93 in der Art der Referenzelektrode 93 auch in anderen Sensoren, etwa Biosensoren, Chemosensoren oder dergleichen eingesetzt werden, in denen ein chloridfreies Referenzelektrodensystem gewünscht ist. Eine Verbesserung der Stabilität kann durch Einsatz von Legierungen statt einem reinem Metall, wie z.B. hier Pb, erzielt werden.

Statt einem elektrochemischen Sensor 92 kann selbstverständlich auch ein anderes Verfahren mit einem anderen Sensortyp eingesetzt werden und etwa eine photometrische Bestimmung des Fremdstoffs 89 durchgeführt werden. Der elektrochemische Sensor 92 wird dabei z.B. durch eine photometrische Detektoreinheit (nicht dargestellt) ersetzt werden. Bei einem beispielhaften Nachweis von S02 im Wein kann im Probenbehälter 80 eine Indikatorlösung (Pararosanilin-Formaldehyd-Mischung bzw. DTNB) oder Sulfitoxidase und NADH vorgelegt und der Blindwert photometrisch bestimmt werden. Danach erfolgt die SO₂-Freisetzung wie später beschreiben, wobei eine Reaktion (Farbreaktion oder Verbrauch an NADH) stattfindet, die photometrisch erfasst und quantifiziert werden kann. Bei gefärbten Proben kann es günstig sein, das Zufügen der Indikatorlösung oder die enzymatische Bestimmung besser nicht in der Probe, sondern durch Separation der Probe und dem Indikator mittels einer semipermeablen Membran, eines Gasstroms oder dergleichen vorzunehmen. Bei wenig gefärbten Proben kann die direkte Bestimmung in der Probe vorgenommen werden.

Das freigesetzte SO₂ wird mit der Pararosanilin-Formaldehyd-Mischung in Verbindung gebracht, wobei nach einer entsprechenden Wartezeit eine Farbreaktion erfolgt. Mittels einer photometrischen Einheit kann die Absorptionsänderung bei einer bestimmten Wellenlänge gemessen und quantifiziert werden. Eine andere Möglichkeit ist eine Farbreaktion mit DTNB (5,5'-Dithio-bis(2-nitrobenzoesäure)). Dabei kommt es bei der Anwesenheit von Sulfit zu einer Spaltung des DTNB zu TNB (2-Nitro-5-mercaptobenzoesäure), welches eine intensiv gelbe Färbung bewirkt, die photometrisch quantifiziert werden kann. Ebenso kann eine enzymatische Detektion eingesetzt werden. Bei der enzymatischen Detektion wird Sulfit-Oxidase eingesetzt. Aus Sulfit entsteht dabei bei Anwesenheit von Sauerstoff und Wasser Sulfat sowie Wasserstoffperoxid, welches wiederum mit NADH (Nicontinamid-Adenin-Dinukleotid) reagiert. Die quantitative Bestimmung erfolgt photometrisch über den Verbrauch an NADH. Dabei kann die Detektoreinheit von der Probe durch eine Membran oder ein Gasleitungssystem abgetrennt werden.

Die Fig. 6a-6c erläutern die Vorbereitung eines bevorzugten Probenbehälters 80 für eine Bestimmung des Gehalts eines Fremdstoffs 89 in einer Matrix 88, insbesondere einer Bestimmung des SO₂-Gehalts in Wein, insbesondere des gesamten Gehalts an gelöstem und gebundenem SO₂.

Gemäß einer bevorzugten Ausgestaltung ist der Probenbehälter 80 vorbefüllt und mit einem Substrat 83 gefüllt, das in im unteren Bereich des Probenbehälters 80 gelagert ist (Fig. 6a), der später einen Mischbereich 82 bildet. Das Substrat 83 ist z.B. ein Gel, Gelatine, Agar-Agar oder dergleichen, welches bei Kontakt mit Wasser sich auflöst und dabei die Lösung verwirbelt und so eine gute Durchmischung der damit in Kontakt kommenden Komponenten auch bei relativ kleinen Probenvolumina führt. Dem Substrat 83 ist vorzugsweise ein Adduktbildner 84 beigefügt. Auf das Substrat 83 kann auch verzichtet werden. Um den Gehalt an gelöstem Fremdstoff 89 zu binden, wird ein Adduktbildner 84, zugegeben. Der Adduktbildner 84 ist ein Bindungspartner für den gelösten Fremdstoff 89. Vorzugsweise wird der gesamte "freie", d.h. gelöste Fremdstoff 89 an den Bindungspartner gebunden, wodurch das Freisetzen des Fremdstoffs 89 aus der Matrix 88 beeinflusst wird, insbesondere verzögert wird. Das Freisetzverhalten von ursprünglich gelöstem und ursprünglich gebundenem Fremdstoff 89 wird aneinander angeglichen. Vorzugsweise ist zur Freisetzung des Fremdstoffs 89 ein saurer pH-Wert eingestellt.

Bei der Bestimmung von SO₂ im Wein wird vorzugsweise ein Bindungspartner, der mindestens eine zur Bisulfit-Addition befähigte Aldehyd- und/oder Ketogruppe aufweist, unmittelbar vor der Messung zum Abbinden des übrigen freien SO₂ und zum Angleichen der Konzentrations- und/oder Zeitprofile zugesetzt. Dies erfolgt vorzugsweise dann, wenn der Gesamtgehalt des Fremdstoffs 89 in gelöster und gebundener Form bestimmt werden soll. Soll nur der Gehalt an gelöstem Fremdstoff 89 bestimmt werden, kann eine Zugabe des Adduktbildners 84 unterbleiben.

Der Adduktbildner 84 kann in fester Form zudosiert werden oder in flüssiger Form als Reinstoff oder Lösung. Vorteilhaft kann der Adduktbildner 84 als Lösung in Probengläschen dosiert, gefriergetrocknet und somit in einen transportfähigen Zustand versetzt werden. Es kann eine genau auf die zuzugebende Menge der Fremdstoff 89 enthaltenden Matrix 88 abgestimmte Menge des Adduktbildners 84 im Probenbehälter 80 gebrauchsfertig für den Anwender bereitgestellt werden.

Alternativ ist zur verzögerten Freisetzung des gelösten Fremdstoffgehalts auch eine Verdeckelung durch eine Sperrschicht denkbar, etwa eine aufschwimmende Schicht mit geringerer spezifischer Dichte als Wasser, z.B. eine Ölschicht, die ein vorzeitiges Austreten des gelösten Fremdstoffs 89 unterbindet. Zur Vermeidung von starker Schaumbildung kann ein Entschäumer zugesetzt werden, z.B. ein siliconölhaltiges Mittel. Ebenso kann eine Verzögerung des Fremdstoff-Übergangs auch durch Polymerbildung in der Probe ermöglicht werden, indem eine polymerbildende Komponente zugesetzt wird, die vorzugsweise unter Säureeinfluss polymerisiert. Denkbar ist auch, das Trägergas nicht durch die Matrix 88 hindurchzuführen, sondern eine permeable Schicht zwischen Trägergas und Matrix 88 anzuordnen, die das Trägergas von der Matrix 88 trennt und den Übergang des Fremdstoffs 89 von der Matrix 88 in das Trägergas im Gasraum 86 erlaubt.

Wie in der Fig. 6a angedeutet, wird eine definierte Menge der zu untersuchenden Matrix 88, die gegebenenfalls Fremdstoff 89 enthalten kann, zudosiert, z.B. mit einer Pipette (Fig. 6b). Die Mengen an Adduktbildner 84 und Matrix 88 sind vorteilhafterweise aufeinander abgestimmt. Gegenüber dem zu erwartenden Gehalt an Fremdstoff 89 ist der Adduktbildner 84 im Überschuss vorhanden.

Die den Fremdstoff 89 enthaltende Matrix 88 vermischt sich mit dem etwaig vorhandenen Substrat 83 und dem Adduktbildner 84 (Fig. 6b), wobei das Substrat 83 sich auflöst und eine Durchmischung der Fremdstoff 89 enthaltenden Matrix 88 und des Adduktbildners 84 stattfindet. Gemäß der Erfindung erfolgt dabei durch den Adduktbildner 84 zunächst ein Abbinden des freien Anteils des Fremdstoffs 89, der in der Matrix 88 gelöst ist (Fig. 6b). Der ursprünglich freie Anteil des Fremdstoffs 89 liegt danach gebunden vor und kann gemeinsam mit dem ursprünglich in der Matrix 88 gebundenen Anteil des Fremdstoffs 89 freigesetzt werden, sobald die Matrix 88 mit dem Reagenz 58 in Kontakt kommt (Fig. 6c).

Durch Zugabe des Reagenz 58 erfolgt ein Aufbrechen der Bindungen, wodurch der Gesamtgehalt an Fremdstoff 89, also der ursprünglich in der Matrix 88 gelöste und der ursprünglich in der Matrix 88 fest gebundene Gehalt, freigesetzt wird und aus dem Gasraum 86 mit einem Trägergas zum Sensorbereich 90 transportiert werden kann. Durch die verzögerte Freisetzung des ursprünglich gelösten Gehalts des Fremdstoffs 89 gleichzeitig mit dem ursprünglich gebundenen Gehalt des Fremdstoffs 89 kann die Messung des Gesamtgehalts an Fremdstoff 89 in der Matrix 88 hinreichend genau erfolgen. Unterschiedliche Konzentrationsprofile für unterschiedlich stark gebundene Fremdstoffe 89 aufgrund der nicht im chemischen Gleichgewicht befindlichen Messung des Fremdstoffgehalts können vorteilhaft vermieden werden.

So ist beim SO₂-Nachweis in Wein mit unterschiedlichen Konzentrationsprofilen der unterschiedlich stark gebundenen sulfithaltigen Spezies zu rechnen. Ohne den bevorzugten erfindungsgemäßen Messablauf würde zunächst ein frühzeitiger Anstieg der Konzentration des als freies SO₂ vorliegenden Sulfits zu beobachten sein, der nach dem Erreichen des Konzentrationsmaximums relativ steil abfällt. Für Spezies, bei denen das chemische Gleichgewicht stark auf der Seite des gebundenen Sulfits liegt, lassen sich Konzentrationsverläufe feststellen, die erst spät ein Maximum erreichen und danach relativ langsam abfallen. Gemäß der Erfindung kann auch bei Vorliegen unterschiedlichster Spezies in nicht bekannten Konzentrationsverhältnissen eine Angleichung des Konzentrationsprofils zur einfachen mathematischen Konzentrationsbestimmung über die Summe aller Sulfitspezies vorgenommen werden.

Zur Bestimmung des Fremdstoffgehalts zur Freisetzung des gebundenen Fremdstoffs 89 ist die Verwendung einer starken Säure als Reagenz 58 günstig, vorzugsweise von mindestens halbkonzentrierter Schwefelsäure.

Dabei kann der Probenbehälter 80 auf eine geeignete Temperatur eingestellt werden. Es kann ein Heiz- und/oder Kühlsystem vorgesehen sein (nicht dargestellt) mit einer Heizeinrichtung und/oder einer Kühleinrichtung sowie einem oder mehreren geeignet angebrachten Temperatursensoren. Alternativ oder zusätzlich kann auch eine Substanz in den Probenbehälter zugegeben werden, die die Reaktion nicht stört, jedoch eine geeignete positive oder negative Lösungsenthalpie aufweist. Negative Lösungsenthalpie bedeutet, dass die Substanz beim Lösen in einem Lösungsmittel, z.B. Wasser, Wärme freisetzt, positive Lösungsenthalpie bedeutet, dass die Substanz beim Lösen Wärme aufnimmt. So kann der Inhalt des Probenbehälters 80 gezielt erwärmt werden oder gezielt abgekühlt werden, ohne dass zusätzliche mechanische oder elektrische Komponenten vorgesehen werden müssen. Das Lösungsmittel kann vorteilhaft im Reagenz 58 und/oder in der Matrix 88 enthalten sein.

Zum Erwärmen kann z.B. gezielt die Hydratationsenergie einer Säure eingesetzt werden, die z.B. als Reagenz 58 zugegeben wird. So kann zur Freisetzung des SO₂ dazu die Hydratationsenergie von H₂SO₄ als Reagenz 58 vorteilhaft ausgenutzt werden. Vorzugsweise wird H₂SO₄ mindestens halbkonzentriert eingesetzt. Dies ist besonders vorteilhaft, wenn der Nachweis nicht nur des gelösten, sondern auch des gebundenen Fremdstoffs 89 erfolgen soll.

Zum Kühlen kann vorteilhaft Ammoniumsulfat zugesetzt werden, das beim Lösen Wärme entzieht. Dies erfolgt vorteilhaft dann, wenn nur der Gehalt des in der Matrix 88 gelösten, aber nicht des gebundenen Fremdstoffs 89 bestimmt werden soll. Der Nachweis des in der Matrix 88 gelösten Fremdstoffs 89 erfolgt bei sanfteren Bedingungen als der Nachweis von in der Matrix 88 gebundenem Fremdstoff 89.

Neben dem Wegfall mechanischer und/oder elektrischer Komponenten gegenüber bekannten Vorrichtungen ist mit dem erfindungsgemäßen Verfahren eine gemeinsame Leitungsführung für Reagenzien möglich, mit denen freier oder gebundener Fremdstoff 89 nachgewiesen werden kann.

Ein Erwärmen des Probenbehälters 80 erfolgt vorzugsweise dann, wenn der Gesamtgehalt des Fremdstoffs 89 in gelöster und gebundener Form bestimmt werden soll. Soll nur der Gehalt an gelöstem Fremdstoff 89 bestimmt werden, wird eine Kühlung des Probenbehälters 80 bevorzugt.

Ein bevorzugter Ablauf einer Messung ist näher im Flussdiagramm in Fig. 7 anhand einer Bestimmung eines Sulfit-Gehalts (SO₂, Fremdstoff 89) in Wein (Matrix 88) beschrieben.

Der Fremdstoffgehalt in der jeweilig zu untersuchenden Matrix, z.B. einer Weinprobe, kann stark variieren. Es kann ein Teil als freies SO₂ vorliegen, das in der Weinmatrix gelöst ist. Ein erheblicher Teil jedoch wird von verschiedenen Komponenten im Wein abgebunden (Bisulfit-Addition an z.B. Aldehyd- oder Ketogruppen). Unterschiedliche Komponenten binden das SO₂ unterschiedlich stark.

Der Aufschluss des gebundenen SO₂ erfolgt mittels einer nichtflüchtigen Säure. Günstig sind z.B. Phosphorsäure oder Schwefelsäure. Die Erwärmung bei Verdünnung ist jedoch bei Schwefelsäure sehr viel stärker ausgeprägt, so dass nur diese ohne zusätzliche Wärmequelle eine vollständige Umsetzung der Bisulfit-Addukte bewirken kann. Zum Einsatz kann z.B. eine etwa 80%ige Schwefelsäure kommen. Es werden z.B. 0,2 ml Probe mit 1,5 ml Säure versetzt. Vorzugsweise wird die Säure in einem Reagenzienbehälter 50 in Form einer vordosierten Ampulle mit Durchstich-Septum eingesetzt, um teure Dosiersysteme zu umgehen.

Hinderlich ist die Korrosionswirkung von Schwefelsäure auf die meisten gängigen Edelstähle, so dass für das Leitungssystem in der Vorrichtung 100 entweder inerte Materialien bevorzugt sind und/oder die Korrosionswirkung der Säure durch geeignete Zusätze vermindert wird. Dies gelingt günstigerweise z.B. durch den Zusatz von Eisensalzen zur Säure. Die Materialauswahl wird durch die technische Anordnung der Reagenzzuführung (z.B. Durchstich-Ampulle) stark eingeschränkt, da hierdurch eine ausreichende Stabilität gefordert wird.

Das freie SO₂ im Wein hingegen wird bei "milden" Reaktionsbedingungen bestimmt. Die bestehenden Bisulfit-Addukte sollen hierdurch nicht beeinträchtigt werden. Zum Austreiben des SO₂ aus der Lösung ist ein saures Milieu notwendig. Als Säure wird vorzugsweise eine nichtflüchtige Säure eingesetzt (z.B. Phosphorsäure). Die Reaktionsbedingungen können durch Abkühlen des Systems noch optimiert werden, z.B. durch Zusatz von Salzen mit positiver Lösungsenthalpie, die ein Abkühlen beim Lösen bewirken oder eine aktive Kühlung durch ein geeignetes Kühlelement.

Die Messroutine beginnt in Schritt 200. In Schritt 202 wird geprüft, ob der Probenbehälter 80 in den Reaktionsbereich 60 und der korrespondierende Reagenzienbehälter 50 in den Reagenzienbereich 40 und der Vorrichtung 100 (Fig. 1) eingestellt wurde. Der Reagenzienbehälter 50 ist mit dem Reagenz 58 gebrauchsfertig befüllt und muss von dem Anwender nur in den Reagenzienbereich 40 eingebracht werden. Der Probenbehälter 80 ist vorzugsweise gegebenenfalls gebrauchsfertig befüllt und wird vom Anwender kurz vor Messbeginn mit einer definierten Menge einer zu untersuchenden Weinprobe versehen. Durch geeignete Codierungen an dem Reagenzienbehälter 50 und/oder dem Probenbehälter 80 kann festgestellt werden, ob der Reagenzienbehälter 50 mit dem richtigen Reagenz 58 für die zu untersuchende Probe im Probenbehälter 80 vorhanden ist.

Falls der Reagenzienbehälter 50 und/oder der Probenbehälter 80 sich nicht am vorgesehenen Platz in der Vorrichtung 100 befinden, oder ein falscher Reagenzeinbehälter 50 eingesetzt wurde, kann die Messroutine nicht gestartet werden und endet in Schritt 238. Gegebenenfalls kann ein Alarmsignal ausgegeben werden und die Fehlfunktion den Anwender angezeigt werden. Das Starten der Messroutine kann beispielsweise ein Startsignal per Knopfdruck, Tastatur-Eingabe, Eingabe über Touchscreen oder dergleichen erfolgen. Das Starsignal kann vor oder nach dem Einstellen des Reagenzienbehälter 50 und der Probenbehälter 80 ausgelöst werden.

Sind der Reagenzienbehälter 50 und der Probenbehälter 80 ordnungsgemäß in die Vorrichtung 100 eingestellt, wird in Schritt 204 geprüft, in welchem Modus gemessen werden soll, d.h. ob nur das freie SO₂ oder der Gesamtgehalt an freiem und gebundenem SO₂ im Wein bestimmt werden soll.

Im Falle einer Messung "Gesamt-SO₂" befindet sich in dem gebrauchsfertig vorbefüllten Probenbehälter 80 ein Adduktbildner 84. Dieser kann gebrauchsfertig mit oder ohne Substrat 83 in dem Probenbehälter 80 vorliegen, bevor die zu untersuchende Weinprobe zugegeben wird. Kurz vor Messbeginn ist eine Weinprobe in definierter Menge in den Probenbehälter 80 zugegeben worden. Während einer kurzen Reaktionszeit von z.B. 0,5 - 2 Minuten wird das im Wein enthaltene freie SO₂ durch den Adduktbildner 84, z.B. ein Natriumsalz der Brenztraubensäure, gebunden. In der Weinprobe befinden sich nunmehr die SO₂-Anteile nur noch in gebundener Form.

Nach ausreichender Reaktionszeit wird in Schritt 222 das Reagenz 58, vorzugsweise H₂SO₄, in den Probenbehälter 80 überführt, indem ein Fördermittel, z.B. die Pumpe 34, ein Trägergas in den Reagenzienbehälter 50 pumpt und das Reagenz 58 aus dem Reagenzeinbehälter 50 in den Probenbehälter 80 gedrückt wird.

Nach einer Reaktionszeit von z.B. 1 Minute, während der der Aufschluss der SO₂-Adukte in die Edukte SO₂ und Adduktbildner 84 erfolgt, wird das freiwerdende SO₂, das nunmehr sowohl die ursprünglich gelösten als auch gebundenen Anteile enthält, mittels eines von der Pumpe 34 gelieferten Trägergasstroms, z.B. Luft, aus dem Probenbehälter 80 ausgetrieben und dem Sensorbereich 90 (Fig. 2) zugeführt. Vorzugsweise wird als Adduktbildner 84 ein Salz der Brenztraubensäure eingesetzt, das den Vorteil hat, dass es in festem Zustand vorliegt und in dieser Form hinreichend chemisch stabil ist. Die Brenztraubensäure weist eine relativ hohe Bindungsaffinität auf und liefert stabile Bisulfit-Addukte. Der Adduktbildner 84 kann als Tablette oder in flüssiger Form als Lösung oder Flüssigkeit mit dem Wein zugegeben werden oder bereits im Probenbehälter 80 in der gewünschten Menge enthalten sein.

In Schritt 230 erfolgt die Datenauswertung, die in Schritt 232 in einer Anzeige z.B. optisch dargestellt werden, wobei in Schritt 234 ein Messprotokoll ausgegeben werden kann.

Soll nur der Gehalt des freien SO₂ gemessen werden, entfällt die erste Reaktionszeit, in der das freie SO₂ gebunden werden soll, da kein Adduktbildner 84 in den Probenbehälter 80 eingeführt wurde bzw. ein konfektionierter Probenbehälter ohne Adduktbildner 84 eingesetzt wurde. Auf Schritt 204 folgt daher Schritt 210, in dem das Reagenz 58 in den Probenbehälter 80 überführt wird. In Schritt 212 treibt ein durch das Reaktionsgemisch strömender Trägergasstrom das SO₂ aus und leitet dieses zur Sensoreinheit 90. Der Nachweis von freiem (gelöstem) SO₂ erfordert nur relativ milde Reaktionsbedingungen. Gegebenenfalls kann eine Kühlung des Probenbehälters 80 erfolgen, um ein unerwünschtes Freisetzen des gebundenen SO₂-Anteils in der Weinprobe bei unerwünscht ansteigender Temperatur zu vermeiden.

In Schritt 230 erfolgt die Datenauswertung, die in Schritt 232 in einer Anzeige optisch dargestellt werden kann, wobei in Schritt 234 ein Messprotokoll ausgegeben werden kann.

Die Auswertung kann theoretisch über mehrere Arten erfolgen:
- numerische Integration der Messkurve (Fläche der Kurve)
- Mittelwert über einen bestimmten Bereich
- Median über einen bestimmten Bereich
- Peakhöhe
- Optionale Baselinekorrektur
- Auswertung der vollständigen Messkurve bis hin zu einem gewissen Rest-SO₂-Gehalt im Trägergas
- Auswertung nur von Teilen der Messkurve (zeitliche Abschnitte oder Abhängigkeit von Signalhöhen bzw. relativen Signalhöhen)
- Auswertung des Signal-Plateaus der Messkurve (z.B. bei einer Zirkularbegasung, bei der das aus dem Probenbehälter 80 abtransportierte Gas im Kreislauf zum Sensor 92 und über den Reagenzienbehälter 50 zurück zum Probenbehälter 80 und Sensor 92 geführt wird).

Nach Ende der Datenauswertung wird der Probenbehälter 80 aus der Vorrichtung 100 entfernt und der Probenkopf gereinigt, z.B. mit einem befeuchteten Tuch abgewischt. Es kann ein Spülgläschen statt des Probenbehälters 80 eingestellt werden, wodurch der Spülvorgang in Schritt 236 automatisch gestartet wird, welcher z.B. 1 Minute andauert und die Messroutine endet in Schritt 238. Im Anschluss wird das Spülgläschen entfernt und der Probenkopf trockengerieben. Der leere Reagenzeinbehälter 50 wird aus der Vorrichtung entfernt.

Im Folgenden sind günstige beispielhafte Parameter für die Messung genannt. Als Säuren für die Bestimmung des SO₂ kommen Schwefelsäure, Phosphorsäure, Salzsäure sowie sämtliche organische oder anorganischen Säuren in Frage, mit denen ein geeigneter pH-Wert erreicht werden kann. Vorzugsweise eignen sich Säuren, die im eingesetzten Konzentrations- und Temperaturbereich wenig bis gar nicht flüchtig sind, oder falls flüchtig, die Detektion des SO₂ hierdurch nicht beeinträchtigen.

Ein gemäß der Erfindung günstiger pH-Bereich für die Bestimmung des gesamten SO₂-Gehalts, d.h. des Gehalts sowohl an gelöstem ("freies") als auch an gebundenem SO₂, liegt bei pH<1 für die Lösung, die in dem Probenbehälter 80 nach der Zuführung des Reagenz 58 vorliegt.

Dies kann z.B. durch etwa 0,1 Mol/L Schwefelsäure in dieser Lösung oder durch etwa achtprozentige Phosphorsäure in dieser Lösung erzielt werden, abhängig von der chemischen Zusammensetzung der Lösung.

Je stärker der pH-Wert abgesenkt wird, d.h. je höher die Säurekonzentration in der Lösung ist, desto leichter erfolgt die Umsetzung des gebundenen SO₂ in gelöstes SO₂.

Ein günstiger pH-Bereich zur Bestimmung des Gehalts an freiem SO₂ liegt zwischen 0,5 und 2, vorzugsweise zwischen 0,8 und 1,5 für die Lösung, die in dem Probenbehälter 80 nach der Zuführung des Reagenz 58 vorliegt. Dies entspricht in etwa einer Konzentration von 0,03- bis 0,2 Mol/L Schwefelsäure in dieser Lösung. Dementsprechend lässt sich auch Phosphorsäure in geeigneter Konzentration verwenden.

Der Protolysebereich der schwefligen Säure ist bekanntermaßen so ausgebildet, dass bei niedrigen pH-Werten (z.B. pH=0) und hohen pH-Werten (z.B. pH=10) das SO₂ fast vollständig in freier Form vorliegt. Um ein vollständiges Freisetzen des gebundenen SO₂ zu gewährleisten, sollte demnach das gesamte SO₂ im stark sauren Bereich bestimmt werden. Möglich ist auch eine Bestimmung alternativ im stark alkalischen Bereich.

Um in der Lösung das molekular vorliegende SO₂ zu erhalten, sollte ein pH-Wert kleiner etwa pH=1,5 vorliegen, da nur diese Form sich in die Gasphase überführen lässt. Um unter diesen Bedingungen eine Bestimmung des Gehalts an gelöstem SO₂ durchführen zu können, muss einer Umsetzung des gebundenen SO₂ entgegengewirkt werden. Hierzu wird vorzugsweise eine niedrige Temperatur gewählt, um eine Rückreaktion der Bisulfit-Addukte zu vermeiden. Dies erlaubt es auch, in niedrigen pH-Bereichen vorzugsweise unterhalb von pH = 1,5, zu arbeiten, um ein Vorliegen des molekularen SO₂ in der Lösung zu erzielen. Zur Bestimmung des Gehalts an gelöstem ist daher eine Temperatur zwischen 0°C und 25°C geeignet.

Um den Gesamtgehalt von SO₂ in der Weinmatrix zu bestimmen, sollte der pH-Wert möglichst niedrig sein. Dies wirkt sich zum einen günstig auf das Vorliegen des molekularen SO₂ aus und ermöglicht zum anderen eine Rückreaktion der Bisulfit-Addition. Für den Nachweis des gebundenen SO₂ sollte ein kleiner pH-Wert von pH<1, vorzugsweise pH<0,5, vorliegen. Eine erhöhte Temperatur kann die Rückreaktion beschleunigen. Somit sind erhöhte Temperaturen und ein stark saures Milieu günstig für die Bestimmung des Gesamtgehalts an SO₂.

Einige bevorzugte Reagenzienzusammensetzungen und Mengen sind im Folgenden genannt. Die Prozentangaben beziehen sich auf Gewichtsprozent.
1) Nachweis des Gesamtgehalts von SO₂ in Wein:
   Probenvolumen an Wein im Probenbehälter 80 z.B. 100-300 µl, vorzugsweise 150-250 µl.

Komponente 1: Adduktbildner (10-100 µl, vorzugsweise 20-50 µl pro Probe) Konzentrierte Lösung des Natriumsalzes der Brenztraubensäure in Wasser. Günstig ist eine konzentrierte Lösung, da diese nur wenig Volumen einnimmt. Es kann jedoch auch eine stärker verdünnte Lösung zum Einsatz kommen.

Komponente 2: Agar-Schicht (100-400 µl, vorzugsweise 150-250 µl pro Probe) 0,3-2,5%, vorzugsweise 0,5-1,5% Agar (Lösung von 0,3-2,5 g, vorzugsweise 0,5-1,5 g Agar in 100 ml Wasser) 5% Na-Salz der Brenztraubensäure

Weniger als 0,3% Agar führt zu wenig stabilen Gelen, eine zu hohe Konzentration führt zu festen Gelen, die weniger gut geeignet sind. Bevorzugt ist ein Bereich von 0,3 bis 2,5%. Die Brenztraubensäure bzw. deren Salz kann ggf. auch weggelassen werden, da eine Zugabe des Adduktbildners (Komponente 1) erfolgt und diese Zugabe nicht durch den Gehalt im Gel ersetzt werden kann.

Komponente 3: Säure (1-3 ml, vorzugsweise 1,25-1,75 ml / Probe) 70-90%ige Schwefelsäure mit ca.0,2-5‰, vorzugsweise 1-1,5‰ Fe(III)-Sulfat Hydrat.

Zu hohe Säurekonzentrationen bereiten technische Probleme und können auch die Weininhaltsstoffe in störender Weise beeinflussen. Geringere Konzentrationen als 60% erfordern eine zusätzliche Heizung. Mit Zusatzheizung kann mit ca. 40%iger Schwefelsäure gearbeitet werden. Zweckmäßigerweise muss auch die Verdünnung durch die Weinmenge (hier: 1,5 ml Reagenz und 200 µl Wein) sowie die anderen Zusatzreagenzien berücksichtigt werden.

Die Probenmenge kann auch erhöht werden, wobei dann zweckmäßigerweise zumindest die Komponenten 1 und 3 im gleichen Verhältnis zur geänderten Probenmenge erhöht werden.
2) Nachweis des Gehalts von freiem SO₂ im Wein:
   Probenvolumen an Wein im Probenbehälter 80 z.B. 100-300 µl, vorzugsweise 150-250 µl

Komponente 1: Säure (1-3 ml, vorzugsweise 1,25-1,75 ml / Probe) 5-20%, vorzugsweise 10-15%ige Phosphorsäure mit ca.0,2-5‰, vorzugsweise 0,6-0,8 ‰ Fe(III)-Sulfat Hydrat

Höhere Säurekonzentrationen setzen zu viel gebundenes SO₂ in Freiheit, zu geringe Konzentrationen ergeben zu niedrige Werte im Vergleich zu Standard-Referenzverfahren. Bevorzugt ist ein Bereich zwischen 5% und 20% für die Säurekonzentration. Es muss wiederum die Verdünnung durch die Weinmenge (hier: 1,5 ml Reagenz und 200 µl Wein) sowie mögliche Zusatzreagenzien berücksichtigt werden. Das Eisensalz wird hier lediglich aus dem Grund zugegeben, dass dasselbe Leitungssystem in der Vorrichtung 100 für beide Varianten (Gesamt-SO₂-Gehalt, Gehalt an freiem SO₂) verwendet wird und somit ein gewisser korrosionsinhibitierender Eisengehalt in der Vorrichtung 100 gewährleistet wird.

Komponente 2: (optional) Ammoniumsulfat (100-500 mg, vorzugsweise 150-250 mg / Probe)

Es kann optional ein Salz, das beim Lösungsvorgang Wärme entzieht, zugegeben werden, vorzugsweise ein Salz, welches in Verbindung mit Säure keine flüchtigen Komponenten freisetzt. Bevorzugt ist Ammoniumsulfat. Bei Ammoniumchlorid kann sich daraus bildende Salzsäure in geringen Mengen durch das Trägergas ausgetrieben werden, bei Ammoniumnitrat können störende Stickoxide entstehen.

Es wird möglichst so viel zugegeben, dass ein ungelöster Rest übrig bleibt. Somit wird immer eine nahezu gesättigte Lösung mit reproduzierbareren Ergebnissen erzielt. Hier spielen Fehler bei der Einwaage keine so große Rolle. Geringere Mengen sind natürlich auch einsetzbar, jedoch sollte hier die Einwaage quantitativ erfolgen.

Die Probe (Matrix 88 mit Fremdstoff 89) kann vorzugsweise mittels einer Dosier-Pipette zugeführt werden, z.B. mit einer Kolbenhubpipette. Es sind jedoch hier zahlreiche andere Varianten wie z.B. Dosierpumpe etc. denkbar. Weiterhin ist auch eine Verdünnung der Probe in dem System denkbar, etwa durch Vorlage von Wasser bzw. verdünnter Säure etc., möglicherweise auch in erhitzter Form, evtl. auch Heißdampf als Trägergas.

Die eingesetzte Software ermöglicht es, eventuelle Abweichungen der "Normalzustände" zu erkennen. Es wird z.B. die Abweichung der Nulllinie registriert, die eine Verunreinigung der Außenluft oder des Systems bedeuten kann. Gegen verunreinigte Außenluft kann beispielsweise ein mit Carbonaten beschichteter Aktivkohlefilter eingesetzt werden. Rein rechnerisch kann der Grundgehalt der Außenluft natürlich auch vom Messergebnis abgezogen werden und somit eine hinreichende Korrektur stattfinden.

Zudem weist die Steuereinheit der Vorrichtung 100 Sicherheitsmerkmale auf, die z.B. das Abschalten der Pumpe 34 bei Fehlbedienung bewirkt oder die eingesetzten Reagenzien anhand eines Strichcodes auf Übereinstimmung mit der gewählten Methode überprüft.

In gewissen Grenzen kann eine Fehleranalyse der aufgezeichneten Messkurve durchgeführt werden, was eine erhöhte Sicherheit der Messergebnisse bedeutet. Auch der Sensor 92 kann auf seine Sensitivität hin überprüft und somit der Zustand des Sensors 92 ermittelt werden.

Die Erfindung ermöglicht vorteilhaft einfache Handhabung durch vordosierte Komponenten und eine rasche Analytik durch kurze Reaktionszeiten. Durch die Erwärmung durch das Reagenz an sich fallen teure Komponenten wie z.B. Heizblock weg. Die eingesetzten Hohlnadeln des Nadelsystems im Reagenzienbereich weisen die notwendige Härte auf, um die Anwendung in Verbindung mit einem Durchstich-Septum zu ermöglichen. Da derartige Systeme in anderen Bereichen häufig eingesetzt werden, sind diese als günstige Materialien erhältlich. Denkbar ist jedoch auch ein Leitungssystem aus inerten Materialien wie z.B. Teflon oder anderen Kunststoffen, Glas, Keramik und dergleichen.

## Patentansprüche

1. Vorrichtung (100) zur Bestimmung eines Gehalts wenigstens eines Fremdstoffs (89) in Form von Sulfit in einer Matrix (88) eines festen oder flüssigen Lebensmittels, umfassend wenigstens
- einen Reagenzienbereich (40) zur Bereitstellung von wenigstens einem Reagenz (58)
- einen Reaktionsbereich (60) mit einem Anschlusssystem (62) zum auswechselbaren Anschließen eines wenigstens die Matrix (88) enthaltenden Probenbehälters (80);
- eine Überführungsleitung (13) zwischen dem Reagenzienbereich (40) und dem Reaktionsbereich (60), mit der das wenigstes eine Reagenz (58) zum Reaktionsbereich (60) führbar ist;
- einen Sensorbereich (90) zum Nachweis des aus der Matrix (88) freigesetzten Fremdstoffs (89);
- eine Trägergasleitung (15) zwischen dem Reaktionsbereich (60) und dem Sensorbereich (90), mit der der freigesetzte Fremdstoff (89) zum Sensorbereich (90) führbar ist; und
- eine Ausgangsleitung (17), durch die der gelöste Fremdstoff (89) vom Sensorbereich (90) nach außen führbar ist,
**dadurch gekennzeichnet,**
- **dass** der Reagenzienbereich (40) eine Aufnahme (41) für einen auswechselbaren Reagenzienbehälter (50) aufweist, wobei ein Einsatz (45) vorgesehen ist, der den Reagenzienbehälter (50) teilweise umschließt, so dass der Reagenzienbehälter (50) mit dem Einsatz (45) in die als Vertiefung ausgebildete Aufnahme (41) des Reagenzienbereichs (40) hineindrückbar ist, um ein Nadelsystem (48) der Vorrichtung (100) in den Reagenzienbehälter (50) einzuführen; und
- **dass** der Reagenzienbereich (40) wenigstens eine Sensoreinrichtung (55) umfasst, die ein Vorhandensein oder ein Nichtvorhandensein des wenigstens einen Reagenzes (58) und/oder Reagenzienbehälters (50) in dem Reagenzienbereich (40) automatisch detektiert, wobei der Reagenzienbehälter (50) gebrauchsfertig mit einer definierten Menge wenigstens eines Reagenzes (58) hergerichtet und an die Aufnahme (41) des Reagenzbereichs (40) auswechselbar anschließbar ist.

2. Vorrichtung nach Anspruch 1, **dadurch gekennzeichnet, dass** das Nadelsystem (48) wenigstens eine eingangsseitige Hohlnadel (42) und eine ausgangsseitige Hohlnadel (44) oder eine Hohlnadel mit einem innenliegenden Schlauch umfasst.

3. Vorrichtung nach Anspruch 1 oder 2, **dadurch gekennzeichnet, dass** die Nadeln des Nadelsystems (48) als Baueinheit ausgebildet sind und die Nadeln in einen Block mit Anschlussmöglichkeiten für ein Fördermittel und mit einer Verbindung zum Reagenzienbereich integriert sind.

4. Vorrichtung nach einem der vorhergehenden Ansprüche, **dadurch gekennzeichnet, dass** der Reaktionsbereich (60) zum Anschließen eines Probenbehälters (80) ein Zuführsystem (62) aufweist, durch welches wenigstens das Reagenz (58) zuführbar und freigesetzter Fremdstoff (89) abführbar ist.

5. Vorrichtung nach einem der vorherigen Ansprüche, **dadurch gekennzeichnet, dass** ein Sensor in einen Gasraum (86) des Probenbehälters (80) einführbar ist.

6. Vorrichtung nach einem der vorherigen Ansprüche, **dadurch gekennzeichnet, dass** der Reaktionsbereich (60) eine Heiz- und/oder Kühleinrichtung aufweist.

7. Vorrichtung nach einem der vorhergehenden Ansprüche, **dadurch gekennzeichnet, dass** der Sensorbereich (90) einen photometrischen Sensor oder einen elektrochemischen Sensor (92) umfasst, insbesondere wobei der elektrochemische Sensor (92) eine Referenzelektrode (93) umfasst mit einem chloridfreien Redoxsystem, vorzugsweise Pb/PbSO₄.

8. Vorrichtung nach einem der vorhergehenden Ansprüche, **dadurch gekennzeichnet, dass** die Trägergasleitung (15) zwischen Reaktionsbereich (60) und Sensorbereich (90) in einen ersten Leitungszweig (15a) zum Zuführen von Trägergas zum Sensorbereich (90) und einen zweiten Leitungszweig (15b) zum Umgehen des Sensorbereichs (90) aufgeteilt ist.

9. Auswechselbarer Reagenzienbehälter (50) für eine Vorrichtung (100) nach einem der vorhergehenden Ansprüche zur Bestimmung eines Gehalts an Fremdstoff (89) in Form von Sulfit in einer Matrix (88) eines festen oder flüssigen Lebensmittels, welcher gebrauchsfertig mit einer definierten Menge wenigstens eines Reagenzes (58) hergerichtet und an die Aufnahme (41) des Reagenzienbereichs (40) verwechslungssicher und auswechselbar anschließbar ist,
- wobei der Reagenzienbehälter (50) in einen Einsatz (45) einsetzbar ist, der den Reagenzienbehälter (50) teilweise umschließt, so dass der Reagenzienbehälter (50) mit dem Einsatz (45) in eine als Vertiefung ausgebildete Aufnahme (41) des Reagenzienbereichs (40) der Vorrichtung (100) hineindrückbar ist, um das Nadelsystem (48) in den Reagenzienbehälter (50) einzuführen,
- wobei das wenigstens eine Reagenz (58) eine Säure oder alkalisch ist, und in Art und Menge genau auf einen vorkonfektionierten Probenbehälters (80) abgestimmt ist, um den Gehalt des Fremdstoffs (89) in der Matrix (88) zu bestimmen.

10. Reagenzienbehälter nach Anspruch 9, **dadurch gekennzeichnet, dass** eine oder mehrere Codierungen (52) zum Nachweis der Anwesenheit in der Vorrichtung (100) und/oder der Art des enthaltenen Reagenzes (58) vorgesehen sind.

11. Reagenzienbehälter nach Anspruch 9 oder 10, **dadurch gekennzeichnet, dass** zusätzlich zu dem Reagenz (58) ein Korrosionsinhibitor enthalten ist.

12. Set aus einem Reagenzienbehälter (50) nach einem der Ansprüche 9 bis 11 und einem Probenbehälter (80) zur Verwendung in einer Vorrichtung (100) nach einem des Ansprüche 1-8 zur Bestimmung eines Gehalts wenigstens eines Fremdstoffs (89) in Form von Sulfit in einer Matrix (88) eines festen oder flüssigen Lebensmittels, wobei
(a) der Probenbehälter (80) einen Adduktbildner (84) in dosierter Menge aufweist, der auf den nachzuweisenden Fremdstoff (89) abgestimmt ist und der zur Bindung eines etwaigen gelösten Fremdstoffs (89) in der Matrix (88) dient; und/oder
(b) der Probenbehälter (80) ein Substrat (83) aufweist, das sich in Kontakt mit einem Reagenz (58) und/oder einer Matrix (88) auflöst; und/oder
(c) der Probenbehälter (80) ein Substrat (83) aufweist, das eine Substanz umfasst, die zur Ausbildung eines Netzwerks befähigt ist, insbesondere eines dreidimensionalen Netzwerks; und/oder
(d) der Probenbehälter (80) eine chemische Komponente mit positiver Lösungsenthalpie oder eine chemische Komponente mit negativer Lösungsenthalpie oder Reaktionsenthalpie enthält; und/oder
(e) der Probenbehälter (80) in seinen Abmessungen auf die Vorrichtung (100) und verwendete Probenmengen und Reagenzmengen in dem Reagenzienbehälter (50) abgestimmt ist; und/oder
(f) der Probenbehälter (80) Abmessungen aufweist, die es erlauben, mit dessen Füllung genau eine Messung zum Nachweis des Fremdstoffs (89) in der Matrix (88) mit abgestimmten Volumina von Matrix (88) und dem Adduktbildner (84) durchzuführen; und/oder
(g) der Adduktbildner (84) als Tablette oder in flüssiger Form mit der Probe zugegeben wird; oder
(h) der Probenbehälter (80) einen Adduktbildner (84) ohne Substrat (83) aufweist.

13. Set aus einem Reagenzienbehälter (50) nach einem der Ansprüche 9 bis 11 und einem Probenbehälter (80) zur Verwendung in einer Vorrichtung (100) nach einem der Ansprüche 1-8 zur Bestimmung eines Gehalts wenigstens eines Fremdstoffs (89) in Form von Sulfit in einer Matrix (88) eines festen oder flüssigen Lebensmittels, wobei
(a) der Probenbehälter (80) ein Substrat (83) aufweist, das sich in Kontakt mit einem Reagenz (58) und/oder einer Matrix (88) auflöst; und/oder
(b) der Probenbehälter (80) ein Substrat (83) aufweist, das eine Substanz umfasst, die zur Ausbildung eines Netzwerks befähigt ist, insbesondere eines dreidimensionalen Netzwerks; und/oder
(c) der Probenbehälter (80) eine chemische Komponente mit positiver Lösungsenthalpie oder eine chemische Komponente mit negativer Lösungsenthalpie oder Reaktionsenthalpie enthält; und/oder
(d) der Probenbehälter (80) in seinen Abmessungen auf die Vorrichtung (100) und verwendete Probenmengen und Reagenzmengen in dem Reagenzienbehälter (50) abgestimmt ist; und/oder
(e) der konfektionierte Probenbehälter (80) ohne Adduktbildner (84) eingesetzt wird.

14. Verfahren zum Betreiben einer Vorrichtung (100) zur Bestimmung eines Gehalts wenigstens eines Fremdstoffs (89) in Form von Sulfit in einer Matrix (88) eines festen oder flüssigen Lebensmittels nach einem der Ansprüche 1 bis 8, **dadurch gekennzeichnet, dass**
- ein ursprünglich gelöster Fremdstoff (89) im Reaktionsbereich (60) in der Matrix (88) zunächst gebunden wird und
- der in der Matrix (88) ursprünglich gelöste Fremdstoff (89) verzögert so freigesetzt wird, dass der ursprünglich gelöste Fremdstoff (89) und etwaiger ursprünglich gebundener Fremdstoff (89) im gleichen Verfahrensschritt gemeinsam aus der Matrix (88) freigesetzt werden.

15. Verfahren nach Anspruch 14, **dadurch gekennzeichnet, dass** zum verzögerten Freisetzen die den Fremdstoff (89) enthaltene Matrix (88) mit einem Adduktbildner (84) vermischt wird, um gelösten Fremdstoff (89) zu binden, und dass nach dem Abbinden des ursprünglich gelösten Fremdstoffs (89) ein Reagenz zugegeben wird, welches den ursprünglich gelösten Fremdstoff (89) und etwaigen ursprünglich gebundenen Fremdstoff (89) aus der Matrix (88) austreibt.

16. Verfahren nach Anspruch 14 oder 15, **dadurch gekennzeichnet, dass** zum verzögerten Freisetzen die den Fremdstoff (89) enthaltene Matrix (88) abgedeckt und/oder eine Polymerisation durchgeführt wird, insbesondere wobei der ausgetriebene Fremdstoff (89) durch ein Trägergas einem Sensor (92) zur Detektion des Fremdstoffs (89) zugeführt wird.

## Claims

1. Device (100) for determining a content of at least one foreign substance (89) in the form of sulphite in a matrix (88) of a solid or liquid food, comprising at least
- one reagent area (40) for providing at least one reagent (58)
- one reaction area (60) with a connection system (62) for interchangeable connection of a sample container (80) at least containing the matrix (88);
- one transfer line (13) between the reagent area (40) and the reaction area (60), with which the at least one reagent (58) can be transferred to the reaction area (60);
- one sensor area (90) for the demonstration of the foreign substance (89) released from the matrix (88);
- one carrier gas line (15) between the reaction area (60) and the sensor area (90), through which the released foreign substance (89) can be transferred to the sensor area (90); and
- one output line (17) through which the dissolved foreign substance (89) can be transferred from the sensor area (90) outwards,
**characterised in**
- **that** the reagent area (40) has a receptacle (41) for an interchangeable reagent container (50), wherein an insert (45) is provided that partially envelops the reagent container (50) so that the reagent container (50) with the insert (45) can be pressed into the receptacle (41), formed as a recess, of the reagent area (40), so that a needle system (48) of the device (100) can be introduced into the reagent container (50); and
- **that** the reagent area (40) comprises at least one sensor device (55) which automatically detects a presence or absence of the at least one reagent (58) and/or reagent container (50) in the reagent area (40), wherein the reagent container (50) is provided ready to use with a defined quantity of at least one reagent (58) and can be interchangeably connected to the receptacle (41) of the reagent area (40).

2. Device according to claim 1, **characterised in that** the needle system (48) has at least one input-side hollow needle (42) and one output-side hollow needle (44) or a hollow needle with an inner tube.

3. Device according to claim 1 or 2, **characterised in that** the needles of the needle system (48) are formed as one module and the needles are integrated in one block with connection means for a transport means and with a connection to the reagent area.

4. Device according to one of the above claims, **characterised in that** the reaction area (60) for connection of a sample container (80) has a feed system (62), through which at least the reagent (58) can be fed and released foreign substance (89) can be discharged.

5. Device according to any one of the above claims, **characterised in that** a sensor can be inserted into a gas space (86) of the sample container (80).

6. Device according to any one of the above claims, **characterised in that** the reaction area (60) has a heating and/or cooling device.

7. Device according to any one of the above claims, **characterised in that** the sensor area (90) comprises a photometric sensor or an electrochemical sensor (92), in particular wherein the electrochemical sensor (92) comprises a reference electrode (93) with a chloride-free redox system, preferably Pb/PbSO₄.

8. Device according to any one of the above claims, **characterised in that** the carrier gas line (15) between the reaction area (60) and sensor area (90) is divided into a first branch line (15a) for supplying carrier gas to the sensor area (90) and a second branch line (15b) for bypassing the sensor area (90).

9. Interchangeable reagent container (50) for a device (100) according to any one of the above claims for determining a content of foreign substance (89) in the form of sulphite in a matrix (88) of a solid or liquid food which is prepared ready for use with a defined quantity of at least one reagent (58), and which is interchangeably connectable to the receptacle (41) of the reagent area (40) without risk of mix-up,
- wherein the reagent container (50) can be inserted into an insert (45) that partially envelops the reagent container (50) so that the reagent container (50) with the insert (45) can be pressed into the receptacle (41), formed as a recess, of the reagent area (40) of the device (100), so that the needle system (48) can be introduced into the reagent container (50).
- wherein the at least one reagent (58) is an acid or alkaline and is matched in its nature and quantity exactly to a pre-prepared sample container (80) to determine the content of foreign substance (89) in the matrix (88).

10. Reagent container according to claim 9, **characterised in that** one or more codings (52) are provided to detect in the device (100) the presence of the contained reagent (58) and/or its nature.

11. Reagent container according to claim 9 or 10, **characterised in that** in addition to the reagent (58), a corrosion inhibitor is contained.

12. Set comprising a reagent container (50) according to any one of claims 9 to 11 and a sample container (80) for use in a device (100) according to any one of claims 1 - 8 to determine the content of at least one foreign substance (89) in the form of sulphite in a matrix (88) of a solid or liquid food, wherein
(a) the sample container (80) contains an adduct former (84) in metered quantity that is matched to the foreign substance (89) to be determined and which serves for binding of any foreign substance (89) dissolved in the matrix (88); and/or
(b) the sample container (80) contains a substrate (83), which dissolves in contact with a reagent (58) and/or a matrix (88); and/or
(c) the sample container (80) contains a substrate (83) which comprises a substance that can form a network, in particular a three-dimensional network; and/or
(d) the sample container (80) contains a chemical component with a positive enthalpy of solution or a chemical component with a negative enthalpy of solution or reaction enthalpy; and/or
(e) the sample container (80) is matched in its dimensions to the device (100) and quantities of sample and reagent used in the reagent container (50); and/or
(f) the sample container (80) has dimensions that allow with its filling to perform exactly one measurement to determine a foreign substance (89) in the matrix (88) with matched volumes of matrix (88) and the adduct former (84); and/or
(g) the adduct former (84) is added as a tablet or in liquid form with the sample; or
(h) the sample container (80) contains an adduct former (84) without substrate (83).

13. Set comprising a reagent container (50) according to any one of claims 9 to 11 and a sample container (80) for use in a device (100) according to any one of claims 1 - 8 to determine the content of at least one foreign substance (89) in the form of sulphite in a matrix (88) of a solid or liquid food, wherein
(a) the sample container (80) contains a substrate (83), which dissolves in contact with a reagent (58) and/or a matrix (88); and/or
(b) the sample container (80) contains a substrate (83) which comprises a substance that can form a network, in particular a three-dimensional network; and/or
(c) the sample container (80) contains a chemical component with a positive enthalpy of solution or a chemical component with negative enthalpy of solution or reaction enthalpy; and/or
(d) the sample container (80) is matched in its dimensions to the device (100) and quantities of sample and reagent used in the reagent container (50); and/or
(e) the pre-prepared sample container (80) is used without adduct former (84).

14. Method for operating a device (100) for determining a content of at least one foreign substance (89) in the form of sulphite in a matrix (88) of a solid or liquid food, according to any one of claims 1 to 8, **characterised in that**
- an originally dissolved foreign substance (89) is first bound in the matrix (88) in reaction area (60) and
- the foreign substance (89) originally dissolved in the matrix (89) is released with a delay such that the originally-dissolved foreign substance (89) and any originally-bound foreign substance (89) are released from the matrix (88) together in the same process step.

15. Method according to claim 14, **characterised in that** for delayed release the matrix (88) containing the foreign substance (89) is mixed with an adduct former (84) to bind dissolved foreign substance (89) and that after binding of the originally-dissolved foreign substance (89), a reagent is added which drives the originally-dissolved foreign substance (89) and any originally-bound foreign substance (89) out of the matrix (88).

16. Method according to claim 14 or 15, **characterised in that** for delayed release the matrix (88) containing the foreign substance (89) is covered and/or a polymerisation is carried out, in particular wherein the driven-out foreign substance (89) is fed by a carrier gas to a sensor (92) for detection of the foreign substance (89).

## Revendications

1. Dispositif (100) pour déterminer une teneur d'au moins une matière étrangère (89) sous forme de sulfite dans une matrice (88) d'une denrée alimentaire solide ou liquide, comprenant au moins
- une zone à réactifs (40) pour mettre à disposition au moins un réactif (58)
- une zone de réaction (60) avec un système de raccordement (62) pour raccorder de manière amovible un récipient d'échantillon (80) contenant au moins la matrice (88) ;
- une conduite de transfert (13) entre la zone à réactifs (40) et la zone de réaction (60), avec laquelle l'au moins un réactif (58) est transférable vers la zone de réaction (60) ;
- une zone de détection (90) pour déceler la matière étrangère (89) libérée depuis la matrice (88) ;
- une conduite de gaz porteur (15) entre la zone de réaction (60) et la zone de détection (90), avec laquelle la matière étrangère libérée (89) est transférable vers la zone de détection (90) ; et
- une conduite de sortie (17), à travers laquelle la matière étrangère dégagée (89) est transférable de la zone de détection (90) vers l'extérieur,
**caractérisé en ce**
- **que** la zone à réactifs (40) présente un logement (41) pour un récipient de réactifs (50) interchangeable, sachant qu'est prévue une cartouche (45) qui entoure partiellement le récipient de réactifs (50) de manière telle que le récipient de réactifs (50) avec la cartouche (45) peuvent être enfoncés dans le logement (41) en forme d'évidement de la zone à réactifs (40) afin d'introduire un système d'aiguilles (48) du dispositif (100) dans le récipient de réactifs (50) ; et
- **que** la zone à réactifs (40) comprend au moins dispositif de détection (55) qui décèle automatiquement une présence ou une absence de l'au moins un réactif (58) et/ou du récipient de réactifs (50) dans la zone à réactifs (40), sachant que le récipient de réactifs (50) est prêt à l'emploi avec une quantité définie d'au moins un réactif (58) et peut être raccordé de manière amovible au logement (41) de la zone à réactifs (40).

2. Dispositif selon la revendication 1, **caractérisé en ce que** le système d'aiguilles (48) comprend au moins une aiguille creuse (42) du côté entrée et une aiguille creuse (44) du côté sortie ou une aiguille creuse avec un tuyau flexible à l'intérieur.

3. Dispositif selon la revendication 1 ou 2, **caractérisé en ce que** les aiguilles du système d'aiguilles (48) sont conçues en tant qu'unité modulaire et que les aiguilles sont intégrées dans un bloc avec possibilités de raccordement pour un moyen d'acheminement et avec une liaison vers la zone à réactifs.

4. Dispositif selon l'une des revendications précédentes, **caractérisé en ce que** pour raccorder un récipient d'échantillon (80), la zone de réaction (60) présente un système d'alimentation (62) à travers lequel au moins le réactif (58) peut être introduit et la matière étrangère libérée (89) peut être évacuée.

5. Dispositif selon l'une des revendications précédentes, **caractérisé en ce qu'**un capteur peut être introduit dans un espace gazeux (86) du récipient d'échantillon (80).

6. Dispositif selon l'une des revendications précédentes, **caractérisé en ce que** la zone de réaction (60) présente un dispositif de chauffage et/ou de refroidissement.

7. Dispositif selon l'une des revendications précédentes, **caractérisé en ce que** la zone de détection (90) comprend un capteur photométrique ou un capteur électrochimique (92), sachant qu'en particulier le capteur électrochimique (92) comprend une électrode de référence (93) avec un système redox exempt de chlorure, de préférence Pb/PbSO4.

8. Dispositif selon l'une des revendications précédentes, **caractérisé en ce que** la conduite de gaz porteur (15) entre la zone de réaction (60) et la zone de détection (90) est divisée en une première branche (15a) destinée à acheminer du gaz porteur vers la zone de détection (90) et une deuxième branche (15b) destinée à contourner la zone de détection (90).

9. Récipient de réactifs interchangeable (50) pour un dispositif (100) selon l'une des revendications précédentes destiné à déterminer une teneur en matière étrangère (89) sous forme de sulfite dans une matrice (88) d'une denrée alimentaire solide ou liquide, lequel récipient est prêt à l'emploi avec une quantité définie d'au moins un réactif (58) et peut être raccordé sans risque de confusion et de manière amovible au logement (41) de la zone à réactifs (40),
- sachant que le récipient de réactifs (50) peut être placé dans une cartouche (45) qui entoure partiellement le récipient de réactifs (50) de manière telle que le récipient de réactifs (50) avec la cartouche (45) peuvent être enfoncés dans un logement (41) en forme d'évidement de la zone à réactifs (40) du dispositif (100) afin d'introduire le système d'aiguilles (48) dans le récipient de réactifs (50),
- et que l'au moins un réactif (58) est un acide ou une solution alcaline, et est par sa nature et sa quantité adapté avec précision à un récipient d'échantillon préconditionné (80) afin de déterminer la teneur de la matière étrangère (89) dans la matrice (88).

10. Récipient de réactifs selon la revendication 9, **caractérisé en ce qu'**un ou plusieurs encodeur(s) (52) est/sont prévu(s) pour déceler dans le dispositif (100) la présence et/ou la nature du réactif (58) contenu.

11. Récipient de réactifs selon la revendication 9 ou 10, **caractérisé en ce qu'**en plus du réactif (58) est contenu un inhibiteur de corrosion.

12. Kit constitué d'un récipient de réactifs (50) selon l'une des revendications 9 à 11 et d'un récipient d'échantillon (80) destinés à être utilisés dans un dispositif (100) selon l'une des revendications 1 à 8 pour déterminer une teneur d'au moins une matière étrangère (89) sous forme de sulfite dans une matrice (88) d'une denrée alimentaire solide ou liquide dans lequel
(a) le récipient d'échantillon (80) présente un formeur de produit d'addition (84) en quantité dosée, qui est adapté à la matière étrangère (89) à détecter et sert à lier une matière étrangère (89) éventuelle dissoute dans la matrice (88) ; et/ou
(b) le récipient d'échantillon (80) présente un substrat (83) qui se dissout au contact avec un réactif (58) et/ou une matrice (88) ; et/ou
(c) le récipient d'échantillon (80) présente un substrat (83) qui contient une substance capable de former un réseau, en particulier un réseau tridimensionnel ; et/ou
(d) le récipient d'échantillon (80) contient un composant chimique avec une enthalpie de dissolution positive ou un composant chimique avec enthalpie de dissolution négative ou enthalpie de réaction ; et/ou
(e) le récipient d'échantillon (80) est dans ses dimensions adapté au dispositif (100) et aux quantités d'échantillon et de réactif utilisées dans le récipient de réactifs (50) ; et/ou
(f) le récipient d'échantillon (80) présente des dimensions qui permettent, avec son contenu, d'effectuer exactement une mesure pour déceler la matière étrangère (89) dans la matrice (88) avec des volumes de matrice (88) adaptés et le formeur de produit d'addition (84) ; et/ou
(g) le formeur de produit d'addition (84) est ajouté sous forme de pastille ou de liquide avec l'échantillon ; ou
(h) le récipient d'échantillon (80) présente un formeur de produit d'addition (84) sans substrat (83).

13. Kit constitué d'un récipient de réactifs (50) selon l'une des revendications 9 à 11 et d'un récipient d'échantillon (80) destinés à être utilisés dans un dispositif (100) selon l'une des revendications 1 à 8 pour déterminer une teneur d'au moins une matière étrangère (89) sous forme de sulfite dans une matrice (88) d'une denrée alimentaire solide ou liquide, dans lequel
(a) le récipient d'échantillon (80) présente un substrat (83) qui se dissout au contact avec un réactif (58) et/ou une matrice (88) ; et/ou
(b) le récipient d'échantillon (80) présente un substrat (83) qui contient une substance capable de former un réseau, en particulier un réseau tridimensionnel ; et/ou
(c) le récipient d'échantillon (80) contient un composant chimique avec une enthalpie de dissolution positive ou un composant chimique avec enthalpie de dissolution négative ou enthalpie de réaction ; et/ou
(d) le récipient d'échantillon (80) est dans ses dimensions adapté au dispositif (100) et aux quantités d'échantillon et de réactif utilisées dans le récipient de réactifs (50) ; et/ou
(e) le récipient d'échantillon (80) conditionné est utilisé sans formeur de produit d'addition (84).

14. Procédé pour mettre en oeuvre un dispositif (100) destiné à déterminer une teneur d'au moins une matière étrangère (89) sous forme de sulfite dans une matrice (88) d'une denrée alimentaire solide ou liquide selon l'une des revendications 1 à 8. **caractérisé en ce que**
- une matière étrangère (89) à l'origine dissoute dans la zone de réaction (60) est tout d'abord liée dans la matrice (88) et
- la matière étrangère (89) à l'origine dissoute dans la matrice (88) est libérée avec une temporisation de telle façon que la matière étrangère (89) à l'origine dissoute et qu'une éventuelle matière étrangère (89) à l'origine liée sont libérées ensemble de la matrice (88) dans une même étape du procédé.

15. Procédé selon la revendication 14, **caractérisé en ce que** pour la libération temporisée, la matrice (88) contenant la matière étrangère (89) est mélangée avec un formeur de produit d'addition (84) afin de lier de la matière étrangère (89) dissoute, et qu'après la liaison de la matière étrangère (89) à l'origine dissoute, est ajouté un réactif qui expulse de la matrice (88) la matière étrangère (89) à l'origine dissoute et une éventuelle matière étrangère (89) à l'origine liée.

16. Procédé selon la revendication 14 ou 15, **caractérisé en ce que** pour la libération temporisée, la matrice (88) contenant la matière étrangère (89) est recouverte et/ou soumise à une polymérisation, sachant qu'en particulier la matière étrangère (89) expulsée est acheminée par un gaz porteur à un capteur (92) destiné à détecter la matière étrangère (89).
